# EUROPEAN PATENT APPLICATION

(11) **EP 3 858 815 A1**
(43) Date of publication of application: **04.08.2021**
(21) Application number: 20305090.1
(22) Date of filing: 31.01.2020
(51) Int. Cl.: C07D 215/38, A61K 31/47, A61P 29/00, A61P 31/18, A61P 35/00

(54) **CO-CRYSTALS AND SALTS OF 8-CHLORO-N-(4-(TRIFLUOROMETHOXY)PHENYL)QUINOLIN-2-AMINE**

(71) Applicant: ABIVAX, 75008 Paris (FR); CENTRE NATIONAL DE LA RECHERCHE SCIENTIFIQUE, 75794 Paris Cedex 16 (FR); UNIVERSITE DE MONTPELLIER, 34090 Montpellier (FR); Institut Curie, 75248 Paris Cedex 05 (FR)
(72) Inventor: The designation of the inventor has not yet been filed
(74) Representative: Nony

(57) **Abstract**

The present invention relates to co-crystals and pharmaceutically acceptable salts of 8-chloro-N-(4-(trifluoromethoxy)phenyl)quinolin-2-amine, pharmaceutical compositions comprising them as well as their use as medicines and more particularly for use in the prevention and/or treatment of inflammatory diseases, diseases caused by viruses and/or cancer or dysplasia. The present invention also concerns methods for the preparation of said co-crystals and said pharmaceutically acceptable salts.

## Description

### FIELD OF THE INVENTION

The present invention relates to the pharmaceutical field and more particularly to novel co-crystals and novel pharmaceutically acceptable salts of 8-chloro-N-(4-(trifluoromethoxy)phenyl)quinolin-2-amine (also named ABX464), processes for their preparation, their use as medicines and in particular for preventing and/or treating of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia. The present invention also concerns pharmaceutical compositions comprising said co-crystals and/or pharmaceutically acceptable salts of 8-chloro-N-(4-(trifluoromethoxy)phenyl)quinolin-2-amine.

### BACKGROUND OF THE INVENTION

WO2010/143169 application describes the preparation and use of compounds, and in particular quinoline derivatives including certain pharmaceutically acceptable salts useful in the treatment of HIV infection. Said application in particular discloses 8-Chloro-N-(4-(trifluoromethoxy)phenyl)quinolin-2-amine also named (8-chloro-quinoline-2-yl)-(4-trifluoromethoxy-phenyl)-amine which is currently under clinical development.

WO2017/158201 application deals with certain mineral acid or sulfonic acid salts of ABX464.

The implementation of new co-crystals and pharmaceutically acceptable salts of ABX464 provides new opportunities to improve the performance of a pharmaceutical product, for example in terms of solubility and controlling of dissolution rate.

### SUMMARY OF THE INVENTION

The inventors have now developed new pharmaceutically acceptable salts and new co-crystals of ABX464.

Thus, the present invention is intended to provide co-crystals and pharmaceutically acceptable salts of 8-chloro-N-(4-(trifluoromethoxy)phenyl)quinolin-2-amine, as well as pharmaceutical compositions comprising said co-crystals and/or pharmaceutically acceptable salts of ABX464 including a solvate and/or a hydrate thereof. Said co-crystals, pharmaceutically acceptable salts of ABX464 and pharmaceutical compositions comprising them can be used as a medicament and more particularly for treating and/or preventing of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia.

The present invention thus provides co-crystals of 8-chloro-N-(4-(trifluoromethoxy)phenyl)quinolin-2-amine which are chosen among:
- 8-Chloro-N-(4-(trifluoromethoxy)phenyl)quinolin-2-amine : L-Proline having a powder X-ray diffractogram displaying peaks expressed as degree 2-Theta angle at 16.5; 20.6; 21.4; and 22.1 (each time ±0.2), and/or having a single endotherm with an onset temperature of 172.0°C (±2°C);
- 8-Chloro-N-(4-(trifluoromethoxy)phenyl)quinolin-2-amine :Gentisic acid having a powder X-ray diffractogram displaying peaks expressed as degree 2-Theta angle at 7.9; 14.0; 15.2; and 25.2 (each time ±0.2), and/or having a single endotherm with an onset temperature of 133.0°C (±2°C);
- 8-Chloro-N-(4-(trifluoromethoxy)phenyl)quinolin-2-amine : Malonic acid having a powder X-ray diffractogram displaying peaks expressed as degree 2-Theta angle at 9.5; 12.2; 15.8; 17.3; 19.7; 22.8; 24.8; and 25.6 (each time ±0.2), and/or having a single endotherm with an onset temperature of 109.0°C (±2°C); and
- 8-Chloro-N-(4-(trifluoromethoxy)phenyl)quinolin-2-amine : 4, 4'-Bipyridine having a powder X-ray diffractogram displaying peaks expressed as degree 2-Theta angle at 12.0; 19.2; 21.2; and 24.3 (each time ±0.2), and/or having a single endotherm with an onset temperature of 127.0°C (±2°C).

The present invention thus also provides a pharmaceutically acceptable salt of 8-chloro-N-(4-(trifluoromethoxy)phenyl)quinolin-2-amine including a solvate and/or a hydrate thereof which is selected from lactate, oleate, oxalate, palmitate, stearate, valerate, pantothenate, picrate, butyrate, malonate, succinate, bitartrate, malate, mandelate, benzoate, edetate, gluceptate, gluconate, lactobionate, salicylate, disalicylate, mucate, pamoate, adipate, alginate, aspartate, camphorate, cyclopentaneproprionate, digluconate, glucoheptonate, heptanoate, hexanoate, laurate, nicotinate, pamoate, pivalate, propionate, undecanoate and the like, phosphate and the like, camphorsulfonate, 2-hydroxyethanesulfonate, estolate, napsylate, esylate, napadisylate, dodecylsulfate and the like, perchloric acid, boric acid, glycerophosphoric acid, nitric acid, persulfuric acid and the like.

Herein provided are also:
- a method for preparing said co-crystals of 8-chloro-N-(4-(trifluoromethoxy)phenyl)quinolin-2-amine as defined in the present invention;
- a method for preparing said pharmaceutically acceptable salts of 8-chloro-N-(4-(trifluoromethoxy)phenyl)quinolin-2-amine including a solvate and/or a hydrate thereof as defined in the present invention;
- pharmaceutical compositions comprising said co-crystals as defined in the present invention and/or said pharmaceutically acceptable salts of ABX464 including a solvate and/or a hydrate thereof as defined in the present invention and at least one pharmaceutically acceptable excipient;
- said pharmaceutically acceptable salt of ABX464 including a solvate and/or a hydrate thereof as defined in the present invention, said co-crystals as defined in the present invention or the pharmaceutical compositions as defined in the present invention for use as a medicine; and
- said pharmaceutically acceptable salt of ABX464 including a solvate and/or a hydrate thereof as defined in the present invention, said co-crystals as defined in the present invention or the pharmaceutical compositions as defined in the present invention for use in the prevention or treatment of cancer, AIDS, an HIV infection, and/or inflammatory diseases.

A co-crystal is a crystal complex composed of at least two neutral molecules bound together in a crystal lattice by non-covalent interactions. The main difference between solvates and co-crystals is related to the physical state of the pure components: if one of the constituents is liquid at ambient temperature, the molecular complex is then a solvate; if all the components are solid at ambient temperature, the complex is then designated by the term " co-crystal". The major difference between a solvate and a co-crystal is the much greater stability of the co-crystals compared to the solvate. A co-crystal is characterised by the method by which it is obtained and by an ordered three-dimensional structure which is demonstrated, for example, by X-ray diffraction diagrams. It is not possible to know a priori whether two given constituents will be able to form a co-crystal having a particular three-dimensional structure or will simply give rise to a juxtaposition of the two powders.

As used herein, the term "pharmaceutically acceptable" refers to those compounds, materials, excipients, carrier, adjuvant, vehicle, compositions or dosage forms which are, within the scope of sound medical judgment, suitable for contact with the tissues of human beings and animals without excessive toxicity, irritation, allergic response or other problem complications commensurate with a reasonable benefit/risk ratio.

In the context of the invention, the term "treating" or "treatment", as used herein, means reversing, alleviating, inhibiting the progress of, or preventing of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia.

The term "preventing", as used herein, means reducing the risk of onset or slowing the occurrence of a given phenomenon, namely in the present invention, of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia. As used herein, *« preventing* » also encompasses « *reducing the likelihood of occurrence* » or « *reducing the likelihood of reoccurrence ».*

A "subject" (including patient) includes mammals, e.g., humans, companion animals (e.g., dogs, cats, birds, and the like), farm animals (e.g., cows, sheep, pigs, horses, fowl, and the like) and laboratory animals (e.g., rats, mice, guinea pigs, birds, and the like).

As used herein, the term "ambient temperature" or "room temperature" refers to a temperature ranging from 15°C to 30°C, more particularly from 18°C to 25°C.

### BRIEF DESCRIPTION OF THE FIGURES

**Figure 1** is a X-ray powder diagram of a co-crystal of 8-Chloro-N-(4-(trifluoromethoxy)phenyl)quinolin-2-amine : L-Proline (see example 1).
**Figure 2** is a X-ray powder diagram of a co-crystal of 8-Chloro-N-(4-(trifluoromethoxy)phenyl)quinolin-2-amine : Gentisic acid (see example 2).
**Figure 3** is a X-ray powder diagram of a co-crystal of 8-Chloro-N-(4-(trifluoromethoxy)phenyl)quinolin-2-amine : Malonic acid (see example 3).
**Figure 4** is a X-ray powder diagram of a co-crystal of 8-Chloro-N-(4-(trifluoromethoxy)phenyl)quinolin-2-amine : 4,4'-Bipyridine (see example 4).
**Figure 5** is a X-ray powder diagram of anhydrous crystalline ABX464 hemi-napadisylate salt (see example 5).
**Figure 6** is a X-ray powder diagram of anhydrous crystalline ABX464 esylate salt (see example 6).
**Figure 7** is a X-ray powder diagram of crystalline hemi-THF (tetrahydrofuran) solvate of ABX464 hemi-napadisylate (see example 7).

### DETAILED DESCRIPTION OF THE INVENTION

### SALTS OF ABX464

As mentioned above, an object of the present invention is a pharmaceutically acceptable salt of 8-chloro-N-(4-(trifluoromethoxy)phenyl)quinolin-2-amine including a solvate and/or a hydrate thereof which is selected from lactate, oleate, oxalate, palmitate, stearate, valerate, pantothenate, picrate, butyrate, malonate, succinate, bitartrate, malate, mandelate, benzoate, edetate, gluceptate, gluconate, lactobionate, salicylate, disalicylate, mucate, pamoate, adipate, alginate, aspartate, camphorate, cyclopentaneproprionate, digluconate, glucoheptonate, heptanoate, hexanoate, laurate, nicotinate, pamoate, pivalate, propionate, undecanoate and the like, phosphate and the like, camphorsulfonate, 2-hydroxyethanesulfonate, estolate, napsylate, esylate, napadisylate, dodecylsulfate and the like, perchloric acid, boric acid, glycerophosphoric acid, nitric acid, persulfuric acid and the like, particularly is selected from esylate and napadisylate, more particularly is selected from anhydrous crystalline ABX464 hemi-napadisylate salt, anhydrous crystalline ABX464 esylate salt, and crystalline hemi-THF solvate of ABX464 hemi-napadisylate.

Thus, according to one embodiment, said pharmaceutically acceptable salt is anhydrous and is selected from anhydrous crystalline ABX464 hemi-napadisylate salt and anhydrous crystalline ABX464 esylate salt.

Thus, according to another embodiment, said pharmaceutically acceptable salt is under the form of a solvate or a hydrate, more particularly a solvate and still more particularly is crystalline hemi-THF (tetrahydrofuran) solvate of ABX464 hemi-napadisylate.

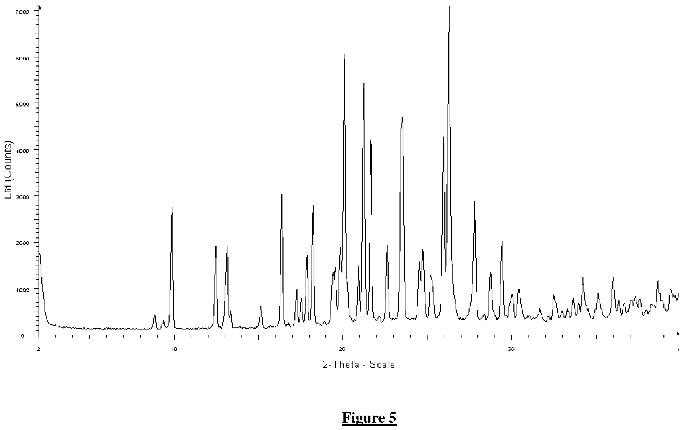

Hence, in a particular embodiment, a pharmaceutically acceptable salt of 8-chloro-N-(4-(trifluoromethoxy)phenyl)quinolin-2-amine as defined in the present invention is selected from anhydrous crystalline ABX464 hemi-napadisylate salt, anhydrous crystalline ABX464 esylate salt, and crystalline hemi-THF solvate of ABX464 hemi-napadisylate, as illustrated respectively in examples 5, 6 and 7 (and respectively figures 5, 6 and 7) of the present text.

The pharmaceutically acceptable salts of 8-chloro-N-(4-(trifluoromethoxy)phenyl)quinolin-2-amine may be for example characterized by X-Ray Powder Diffraction (XRPD) and by Differential Scanning Calorimetry (DSC).

More particularly, anhydrous crystalline ABX464 hemi-napadisylate salt of example 5 has a powder X-ray diffractogram displaying peaks expressed as degree 2-Theta angle at 9.8; 16.4; 18.2; 20.1; 21.2; 21.6; 23.5 and 26.3 (each time ±0.2), and optionally further shows the following additional peaks expressed as degree 2-Theta angle: 12.4; 13.1; 17.8; 20.9; 22.6; 24.5; 24.7; 25.2; and 25.9 (each time ±0.2); and even optionally further the following additional peaks expressed as degree 2-Theta angle: 8.8; 13.3; 15.1; 17.2; 17.5; 19.4; 19.5; and 19.8 (each time ±0.2), as illustrated in figure 5 (powder X-ray diffractogram) and/or has a single endotherm with an onset temperature of 269.0°C (±2°C).

A characteristic X-ray powder diffractogram of an anhydrous crystalline ABX464 hemi-napadisylate salt can be given in figure 5 and its characteristic signals are summarized in the following table 1 :

**Table 1**

| **Angle (2-Theta)** (each time ±0.2) | **Relative intensity (%)** |
|---|---|
| 8.8 | 6 |
| 9.8 | 38 |
| 12.4 | 27 |
| 13.1 | 27 |
| 13.3 | 7 |
| 15.1 | 8 |
| 16.4 | 42 |
| 17.2 | 13 |
| 17.5 | 11 |
| 17.8 | 24 |
| 18.2 | 39 |
| 19.4 | 20 |
| 19.5 | 20 |
| 19.8 | 26 |
| 20.1 | 85 |
| 20.9 | 21 |
| 21.2 | 76 |
| 21.6 | 59 |
| 22.6 | 27 |
| 23.5 | 66 |
| 24.5 | 22 |
| 24.7 | 25 |
| 25.2 | 18 |
| 25.9 | 60 |
| 26.3 | 100 |

According to one embodiment, the anhydrous crystalline ABX464 hemi-napadisylate salt of the present invention presents a powder X-ray diffractogram displaying at least one peak expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least two peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least three peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least four peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least five peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least six peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least seven peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least eight peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least nine peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least ten peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least eleven peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least twelve peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least thirteen peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least fourteen peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention presents a powder X-ray diffractogram displaying at least fifteen peaks expressed as degree 2-Theta angle selected from 9.8; 16.4; 18.2; 20.1; 21.2; 21.6; 23.5; 26.3; 12.4; 13.1; 17.8; 20.9; 22.6; 24.5; 24.7; 25.2; 25.9; 8.8; 13.3; 15.1; 17.2; 17.5; 19.4; 19.5; and 19.8 (each time ±0.2).

According to another embodiment, the anhydrous crystalline ABX464 hemi-napadisylate salt of the present invention has an XRPD substantially similar to that depicted in figure 5.

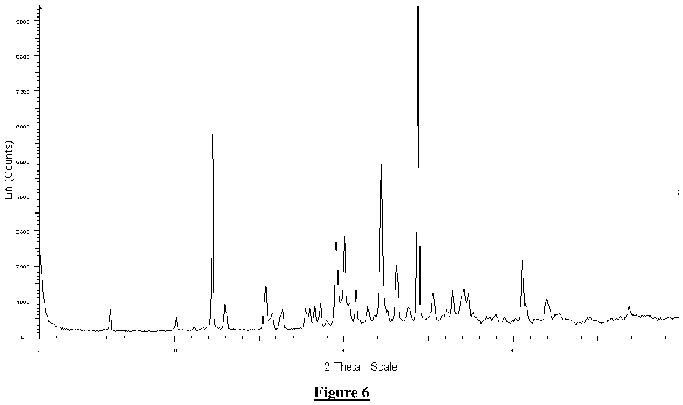

More particularly, anhydrous crystalline ABX464 esylate salt of example 6 has a powder X-ray diffractogram displaying peaks expressed as degree 2-Theta angle at 12.2; and 22.2 (each time ±0.2), and optionally further shows the following additional peaks expressed as degree 2-Theta angle: 6.2; 12.9; 13.1; 15.3; 16.3; 18.2; 18.6; 19.5; 20.0; and 20.7 (each time ±0.2); and even optionally further the following additional peaks expressed as degree 2-Theta angle: 10.1; 15.8; 17.7; 17.9; 20.3; and 21.4 (each time ±0.2), as illustrated in figure 6 (powder X-ray diffractogram) and / or has a single endotherm with an onset temperature of 108.0°C (±2°C).

A characteristic X-ray powder diffractogram of an anhydrous crystalline ABX464 esylate salt can be given in figure 6 and its characteristic signals are summarized in the following table 2 :

**Table 2**

| **Angle (2-Theta)** (each time ±0.2) | **Relative intensity (%)** |
|---|---|
| 6.2 | 13 |
| 10.1 | 9 |
| 12.2 | 100 |
| 12.9 | 17 |
| 13.1 | 11 |
| 15.3 | 27 |
| 15.8 | 11 |
| 16.3 | 13 |
| 17.7 | 14 |
| 17.9 | 14 |
| 18.2 | 16 |
| 18.6 | 15 |
| 19.5 | 47 |
| 20.0 | 49 |
| 20.3 | 15 |
| 20.7 | 23 |
| 21.4 | 15 |
| 22.2 | 85 |

According to one embodiment, the anhydrous crystalline ABX464 esylate salt of the present invention presents a powder X-ray diffractogram displaying at least one peak expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least two peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least three peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least four peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least five peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least six peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least seven peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least eight peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least nine peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least ten peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least eleven peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least twelve peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least thirteen peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least fourteen peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention presents a powder X-ray diffractogram displaying at least fifteen peaks expressed as degree 2-Theta angle selected from 12.2; 22.2; 6.2; 12.9; 13.1; 15.3; 16.3; 18.2; 18.6; 19.5; 20.0; 20.7; 10.1; 15.8; 17.7; 17.9; 20.3; and 21.4 (each time ±0.2).

According to another embodiment, the anhydrous crystalline ABX464 esylate salt of the present invention has an XRPD substantially similar to that depicted in figure 6.

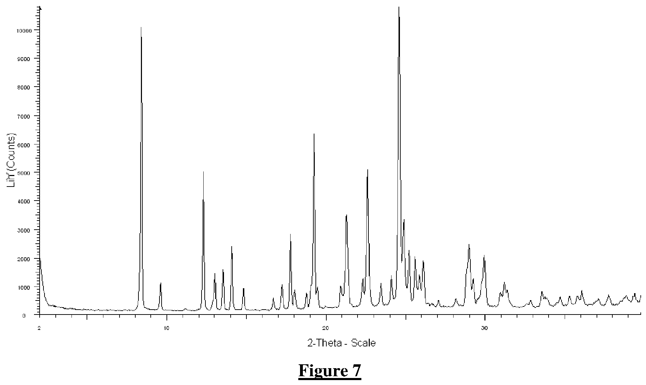

More particularly, crystalline hemi-THF solvate of ABX464 hemi-napadisylate salt of example 7 has a powder X-ray diffractogram displaying peaks expressed as degree 2-Theta angle at 8.4; 12.3; 14.0; 19.2; 21.3; 22.6 and 24.6 (each time ±0.2), and optionally further shows the following additional peaks expressed as degree 2-Theta angle: 9.6; 13.0; 13.5; 14.8; 17.2; 17.8; 23.4; 24.1; 24.9 and 25.2 (each time ±0.2); and even optionally further the following additional peaks expressed as degree 2-Theta angle: 16.7; 18.1; 18.8; 19.5; 20.9 and 22.3 (each time ±0.2), as illustrated in figure 7 (powder X-ray diffractogram) and/ or has a single endotherm with an onset temperature of 172.0°C (±2°C).

A characteristic X-ray powder diffractogram of a crystalline hemi-THF solvate of ABX464 hemi-napadisylate salt can be given in figure 7 and its characteristic signals are summarized in the following table 3 :

**Table 3**

| **Angle (2-Theta)** (each time ±0.2) | **Relative intensity (%)** |
|---|---|
| 8.4 | 93 |
| 9.6 | 10 |
| 12.3 | 46 |
| 13.0 | 13 |
| 13.5 | 14 |
| 14.0 | 22 |
| 14.8 | 9 |
| 16.7 | 5 |
| 17.2 | 10 |
| 17.8 | 26 |
| 18.1 | 8 |
| 18.8 | 7 |
| 19.2 | 59 |
| 19.5 | 9 |
| 20.9 | 9 |
| 21.3 | 32 |
| 22.3 | 12 |
| 22.6 | 47 |
| 23.4 | 10 |
| 24.1 | 13 |
| 24.6 | 100 |
| 24.9 | 31 |
| 25.2 | 21 |

According to one embodiment, the crystalline hemi-THF solvate of ABX464 hemi-napadisylate salt of the present invention presents a powder X-ray diffractogram displaying at least one peak expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least two peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least three peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least four peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least five peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least six peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least seven peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least eight peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least nine peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least ten peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least eleven peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least twelve peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least thirteen peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least fourteen peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention presents a powder X-ray diffractogram displaying at least fifteen peaks expressed as degree 2-Theta angle selected from 8.4; 12.3; 14.0; 19.2; 21.3; 22.6; 24.6; 9.6; 13.0; 13.5; 14.8; 17.2; 17.8; 23.4; 24.1; 24.9; 25.2; 16.7; 18.1; 18.8; 19.5; 20.9 and 22.3 (each time ±0.2).

According to another embodiment, the crystalline hemi-THF solvate of ABX464 hemi-napadisylate salt of the present invention has an XRPD substantially similar to that depicted in figure 7.

### METHOD FOR PREPARING A PHARMACEUTICALLY ACCEPTABLE SALT OF ABX464

Herein is further provided a method for preparing a pharmaceutically acceptable salt of ABX464 (including solvate or hydrate thereof) which comprises the following steps:
a) dissolving ABX464 in a solvent or in a mixture of solvents;
b) adding to the thus obtained mixture of step a) a counter ion under the form of an acid which may be itself already dissolved in a solvent or in a mixture of solvents so as to obtain a ABX464 : counter ion molar ratio comprised between 3:1 and 1:2, particularly between 5:2 and 1:2; more particularly between 2:1 and 1:2, and still more particularly is of 2:1 or 1 :1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent or a mixture of solvents,
e) applying a temperature program;
f) optionally filtrating; and
g) then optionally drying at a temperature comprised between room temperature and 60°C in order to obtain the desired salt of ABX464.

According to one embodiment, the solvent(s) used in step a), step b) and step d) is(are) any solvent conventionally used in crystallization step, particularly is(are) organic solvents, more particularly is(are) selected from a C₁-C₆ aliphatic alcohol, methyl ethyl ketone (also named butanone or MEK), cyclohexane, alkane such as heptane, methylene chloride, chloroform, formic acid, DMSO, 1-methyl-2-pyrrolidone, acetone, acetonitrile, tetrahydrofuran (THF), diethyl ether, dioxane, toluene, ethyl acetate, optionally in admixture with water, and mixtures thereof, still more particularly selected from a C₁-C₆ aliphatic alcohol, a mixture of H₂O/ C₁-C₆ aliphatic alcohol, and mixtures thereof, even more particularly selected from methanol, ethanol, isopropanol, H₂O/methanol, H₂O/ethanol, and mixtures thereof.

The skilled person would know how to determine the more appropriate solvent(s) in each step a), b) and d) so as to obtain the desired pharmaceutically acceptable salt.

According to one embodiment, the solvent(s) used in step a), step b) and/or step d) is(are) the same.

According to another embodiment, the solvent(s) used in step a), step b) and/or step d) is(are) different.

Advantageously, the counter ion under the form of an acid of step b) is ethane sulfonic acid or naphthalene 1,5-disulfonic acid.

Thus, in some embodiments, the solvent of step a), step b) and/or step d) is any solvent conventionally used in the crystallization step, particularly is organic solvents, more particularly is selected from a C₁-C₆ aliphatic alcohol, methyl ethyl ketone (also named butanone or MEK), cyclohexane, alkane such as heptane, methylene chloride, chloroform, formic acid, DMSO, 1-methyl-2-pyrrolidone, acetone, acetonitrile, tetrahydrofuran (THF), diethyl ether, dioxane, toluene, ethyl acetate, optionally in admixture with water, and mixtures thereof, still more particularly selected from a C₁-C₆ aliphatic alcohol, a mixture of H₂O/ C₁-C₆ aliphatic alcohol, and mixtures thereof, even more particularly selected from methanol, ethanol, isopropanol, H₂O /methanol, H₂O /ethanol, and mixtures thereof; and/or the counter ion under the form of an acid of step b) is ethane sulfonic acid or naphthalene 1,5-disulfonic acid.

According to one embodiment, the evaporating step c) is carried out under an inert gas such as N_{2.}

According to one embodiment the step e) relative to the temperature program includes a heating phase at reflux to a temperature comprised between room temperature and the boiling point of the solvent(s), particularly between room temperature and 60°C.

According to one embodiment the step e) relative to the temperature program includes a cooling phase at reflux to a temperature comprised between 0°C and 60°C, particularly between 5°C and 40°C, more particularly between room temperature and 40°C at a rate comprised between 30°C/min and 0.05°C/min, particularly between 10°C/min and 0.05°C/min, more particularly between 5°C/min and 0.05°C/min.

According to one embodiment, the step e) relative to the temperature program includes i) a heating at reflux to a temperature comprised between room temperature and the boiling point of the solvent(s), particularly between room temperature and 60°C and / or ii) a cooling at reflux to a temperature comprised between 0°C and 60°C, particularly between 5°C and 40°C, more particularly between room temperature and 40°C at a rate comprised between 30°C/min and 0.05°C/min, particularly between 10°C/min and 0.05°C/min, more particularly between 5°C/min and 0.05°C/min.

According to a particular embodiment, the step e) relative to the temperature program includes i) a heating at reflux to a temperature comprised between room temperature and 60°C and ii) a cooling at reflux to a temperature comprised between 5°C and 40°C, at a rate comprised between 30°C/min and 0.05°C/min, particularly between 10°C/min and 0.05°C/min, more particularly between 5°C/min and 0.05°C/min.

According to one embodiment, the step f) regarding the filtration is carried out using conventional glass fibers, conventional cellulosic filter papers, PTFE (polytetrafluoroethylene), or PVDF (polyvinylidene fluoride), particularly cellulosic filter paper with 0.45µm or 0.2µm filtration mesh.

According to one embodiment, the step g) regarding the drying is carried out under vacuum at a temperature comprised between 30°C and 60°C, particularly at 40°C.

According to one embodiment, the step g) regarding the drying is carried out under ambient atmosphere at a temperature comprised between 30°C and 60°C, particularly at 40°C.

It is understood that for the preparation of a pharmaceutically acceptable salt of ABX464 as defined in the present invention, 8-chloro-N-(4-(trifluoromethoxy)phenyl)quinolin-2-amine can be previously obtained either from a process as the one described in WO2010/143169 or from any other appropriate process.

According to some embodiments, the method for preparing a pharmaceutically acceptable salt of ABX464 (including solvate or hydrate thereof) comprises the following steps:
a) dissolving ABX464 in a solvent or in a mixture of solvents;
b) adding to the thus obtained mixture of step a) a counter ion under the form of an acid which may be itself already dissolved in a solvent or in a mixture of solvents so as to obtain a ABX464 : counter ion molar ratio comprised between 3:1 and 1:2, particularly between 5:2 and 1:2; more particularly between 2:1 and 1:2, and still more particularly is of 2:1 or 1 :1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent or a mixture of solvents,
e) applying a temperature program which includes i) a heating at reflux to a temperature comprised between room temperature and the boiling point of the solvent(s), particularly between room temperature and 60°C and / or ii) a cooling at reflux to a temperature comprised between 0°C and 60°C, particularly between 5°C and 40°C, more particularly between room temperature and 40°C at a rate comprised between 30°C/min and 0.05°C/min, particularly between 10°C/min and 0.05°C/min, more particularly between 5°C/min and 0.05°C/min.;
f) optionally filtrating by using conventional glass fibers, conventional cellulosic filter papers, PTFE (polytetrafluoroethylene), or PVDF (polyvinylidene fluoride), particularly cellulosic filter paper with 0.45µm or 0.2µm filtration mesh; and
g) then optionally drying under vacuum at a temperature comprised between 30°C and 60°C, particularly at 40°C or under ambient atmosphere at a temperature comprised between 30°C and 60°C, particularly at 40°C in order to obtain the desired salt of ABX464.

According to some embodiments, the method for preparing a pharmaceutically acceptable salt of ABX 464 comprises the following steps:
a) dissolving ABX464 in methanol;
b) adding to the thus obtained mixture of step a) a counter ion which is ethane sulfonic acid or naphthalene sulfonic acid which is itself already dissolved in water/ethanol or ethanol so as to obtain a ABX464 : counter ion molar ratio comprised between 3:1 and 1:2, particularly between 5:2 and 1:2; more particularly between 2:1 and 1:2, and still more particularly is of 2:1 or 1:1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent selected from ethyl acetate, THF, acetone, and mixtures thereof,
e) applying a temperature program which includes (i) a heating phase at reflux to a temperature comprised between room temperature and the boiling point of the solvent(), particularly between room temperature and 60°C and / or (ii) a cooling phase at reflux to a temperature comprised between 0°C and 60°C, particularly between 5°C and 40°C, more particularly between room temperature and 40°C at a rate comprised between 30°C/min and 0.05°C/min, particularly between 10°C/min and 0.05°C/min, more particularly between 5°C/min and 0.05°C/min;
f) optionally filtrating; and
g) then optionally drying at a temperature comprised between room temperature and 60°C in order to obtain the desired salt of ABX464.

According to some embodiments, the method for preparing a pharmaceutically acceptable salt of ABX 464 comprises the following steps:
a) dissolving ABX464 in methanol;
b) adding to the thus obtained mixture of step a) a counter ion which is ethane sulfonic acid or naphthalene sulfonic acid which is itself already dissolved in water/ethanol or ethanol so as to obtain a ABX464 : counter ion molar ratio comprised between 3:1 and 1:2, particularly between 5:2 and 1:2; more particularly between 2:1 and 1:2, and still more particularly is of 2:1 or 1:1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent selected from ethyl acetate, THF, acetone, and mixtures thereof,
e) applying a temperature program which includes (i) a heating phase at reflux to a temperature of 60°C and / or (ii) a cooling phase at reflux to a temperature of 5°C and 40°C, more particularly between room temperature and 40°C at a rate of 1°C/min;
f) optionally filtrating; and
g) then optionally drying at 40°C in order to obtain the desired salt of ABX464.

According to one particular embodiment, ABX464 is dissolved in a C₁-C₆ aliphatic alcohol, in particular methanol, then to this mixture is added naphthalene 1,5-disulfonic acid which is itself already dissolved in a C₁-C₆ aliphatic alcohol, in particular ethanol so as to obtain a ABX464 : counter ion molar ratio comprised between 3:1 and 1:2, particularly between 5:2 and 1:2; more particularly between 2:1 and 1:2, and still more particularly is of 2:1 or 1 :1. Then evaporation under N2 at room temperature (25°C), then addition of acetone, then applying a temperature program before filtration on 0.2µm, and drying on vacuum at 40°C are carried out to obtain anhydrous crystalline ABX464 hemi-napadisylate salt.

According to another particular embodiment, ABX464 is dissolved in a C₁-C₆ aliphatic alcohol, in particular methanol, then to this mixture is added ethane sulfonic acid which is itself already dissolved in a mixture H₂O/ C₁-C₆ aliphatic alcohol, in particular water/ethanol so as to obtain a ABX464 : counter ion molar ratio between 3:1 and 1:2, particularly between 5:2 and 1:2; more particularly between 2:1 and 1:2, and still more particularly is of 1:1. Then evaporation under N2 at room temperature (25°C), then addition of ethyl acetate, then applying a temperature program before evaporation carried out under N2 at room temperature (25°C) are carried out to obtain anhydrous crystalline ABX464 esylate salt.

According to another embodiment, ABX464 is dissolved in a C₁-C₆ aliphatic alcohol, in particular methanol, then to this mixture is added naphthalene1,5-disulfonic acid which is itself already dissolved in a C₁-C₆ aliphatic alcohol, in particular ethanol so as to obtain a ABX464 : counter ion molar ratio between 3:1 and 1:2, particularly between 5:2 and 1:2; more particularly between 2:1 and 1:2, and still more particularly is of 2:1. Then evaporation under N2 at 40°C, then addition of THF, then a temperature program is applied before filtration on 0.2µm, and drying on vacuum at 40°C are carried out to obtain crystalline hemi-THF solvate of ABX464 hemi-napadisylate.

### CO-CRYSTALS OF ABX464

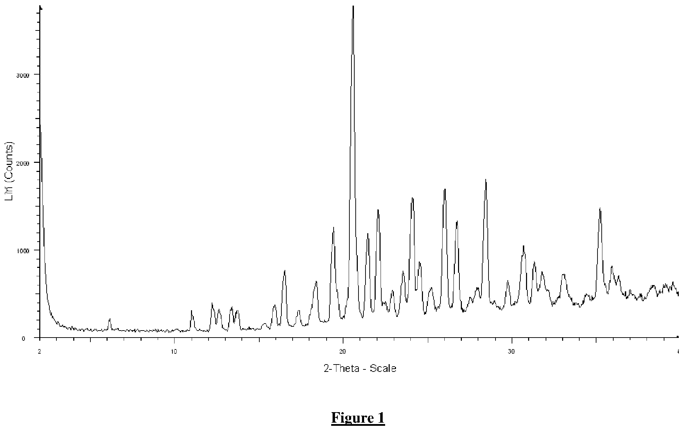

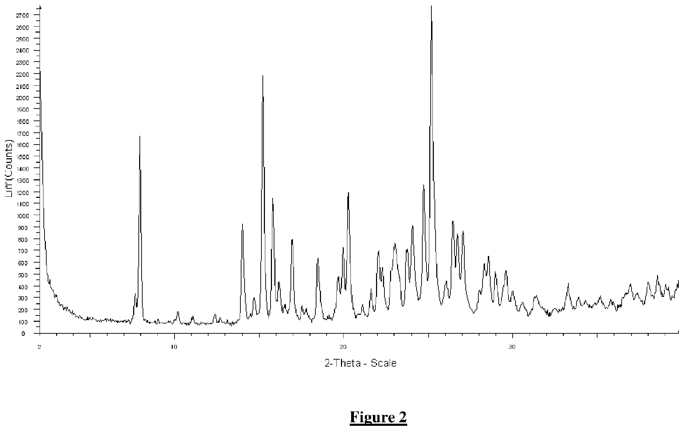

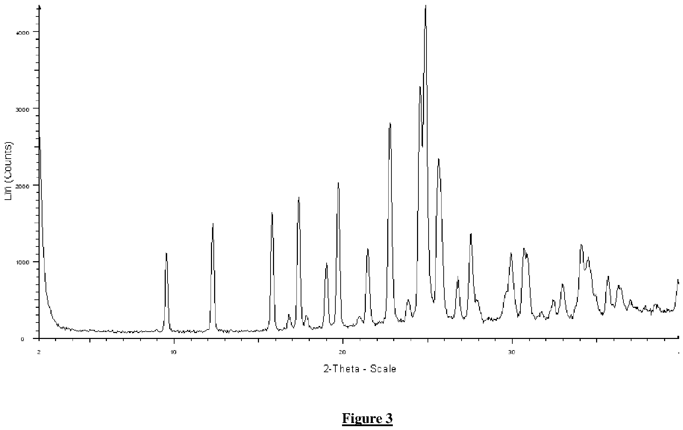

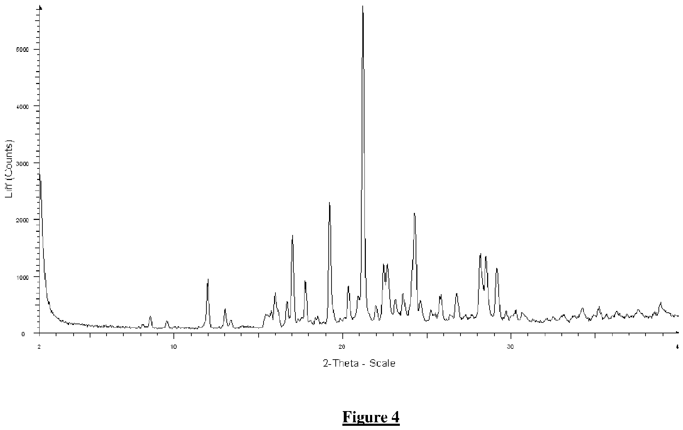

As mentioned above, in another aspect, the present invention relates to co-crystals of 8-chloro-N-(4-(trifluoromethoxy)phenyl)quinolin-2-amine which are chosen among:
- 8-Chloro-N-(4-(trifluoromethoxy)phenyl)quinolin-2-amine : L-Proline having a powder X-ray diffractogram displaying peaks expressed as degree 2-Theta angle at 16.5; 20.6; 21.4; and 22.1 (each time ±0.2), and which may optionally further show the following additional peaks expressed as degree 2-Theta angle: 11.0; 15.9; 18.3; and 19.4 (each time ±0.2); and even optionally further the following additional peaks expressed as degree 2-Theta angle 6.1; 12.2; 12.6; 13.3; 13.7; 15.4; 17.3 and 22.4 (each time ±0.2), optionally further characterized by a powder X-ray diffractogram as illustrated in figure 1 and/or having a single endotherm with an onset temperature of 172.0°C (±2°C);
- 8-Chloro-N-(4-(trifluoromethoxy)phenyl)quinolin-2-amine :Gentisic acid having a powder X-ray diffractogram displaying peaks expressed as degree 2-Theta angle at 7.9; 14.0; 15.2; and 25.2 (each time ±0.2), and which may optionally further show the following additional peaks expressed as degree 2-Theta angle: 15.8; 16.9; 18.5; 19.9; 20.3; 23.0 and 24.7 (each time ±0.2); and even optionally further the following additional peaks expressed as degree 2-Theta angle: 7.6; 14.7; 16.1; 19.7; 21.6; 22.0; 22.3; 23.7; and 24.0 (each time ±0.2), optionally further characterized by a powder X-ray diffractogram as illustrated in figure 2 and/or having a single endotherm with an onset temperature of 133.0°C (±2°C);
- 8-Chloro-N-(4-(trifluoromethoxy)phenyl)quinolin-2-amine : Malonic acid having a powder X-ray diffractogram displaying peaks expressed as degree 2-Theta angle at 9.5; 12.2; 15.8; 17.3; 19.7; 22.8; 24.8; and 25.6 (each time ±0.2), and which may optionally further show the following additional peaks expressed as degree 2-Theta angle: 19.0; 21.4; 24.6; 26.8; 27.6; and 29.9 (each time ±0.2); and even optionally further the following additional peaks expressed as degree 2-Theta angle 16.8; 17.8; 20.9; 23.8; 28.0; and 29.6 (each time ±0.2), optionally further characterized by a powder X-ray diffractogram as illustrated in figure 3 and/or having a single endotherm with an onset temperature of 109.0°C (±2°C); and
- 8-Chloro-N-(4-(trifluoromethoxy)phenyl)quinolin-2-amine : 4, 4'-Bipyridine having a powder X-ray diffractogram displaying peaks expressed as degree 2-Theta angle at 12.0; 19.2; 21.2; and 24.3 (each time ±0.2), and which may optionally further show the following additional peaks expressed as degree 2-Theta angle: 16.0; 17.0; 17.8; 20.3; 22.5; and 22.7 (each time ±0.2); and even optionally further the following additional peaks expressed as degree 2-Theta angle: 8.5; 13.0; 15.7; 16.7; 20.9; 22.0; 23.1; 23.6 and 24.7 (each time ±0.2), optionally further characterized by a powder X-ray diffractogram as illustrated in figure 4 and/or having a single endotherm with an onset temperature of 127.0°C (±2°C).

Said four co-crystals are respectively illustrated in examples 1 (and figure 1), 2 (and figure 2), 3 (and figure 3) and 4 (and figure 4) of the present text.

A characteristic X-ray powder diffractogram of a co-crystal of 8-chloro-N-(4-(trifluoromethoxy)phenyl)quinolin-2-amine : L-Proline can be given in figure 1 and its characteristic signals are summarized in the following table 4 :

**Table 4**

| **Angle (2-Theta)** (each time ±0.2) | **Relative intensity (%)** |
|---|---|
| 6.1 | 5 |
| 11.0 | 8 |
| 12.2 | 10 |
| 12.6 | 8 |
| 13.3 | 9 |
| 13.7 | 8 |
| 15.4 | 4 |
| 15.9 | 9 |
| 16.5 | 20 |
| 17.3 | 8 |
| 18.3 | 17 |
| 19.4 | 33 |
| 20.6 | 100 |
| 21.4 | 31 |
| 22.1 | 38 |
| 22.4 | 10 |

According to one embodiment, the co-crystal of 8-Chloro-N-(4-(trifluoromethoxy)phenyl)quinolin-2-amine : L-Proline of the present invention presents a powder X-ray diffractogram displaying at least one peak expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least two peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least three peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least four peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least five peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least six peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least seven peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least eight peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least nine peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least ten peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least eleven peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least twelve peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least thirteen peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least fourteen peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention presents a powder X-ray diffractogram displaying at least fifteen peaks expressed as degree 2-Theta angle selected from 16.5; 20.6; 21.4; 22.1; 11.0; 15.9; 18.3; 19.4; 6.1; 12.2; 12.6; 13.3; 13.7; 15.4; 17.3 and 22.4 (each time ±0.2).

According to another embodiment, the co-crystal of 8-Chloro-N-(4-(trifluoromethoxy)phenyl)quinolin-2-amine : L-Proline of the present invention has an XRPD substantially similar to that depicted in figure 1.

A characteristic X-ray powder diffractogram of a co-crystal of 8-chloro-N-(4-(trifluoromethoxy)phenyl)quinolin-2-amine : Gentisic acid can be given in figure 2 and its characteristic signals are summarized in the following table 5 :

**Table 5**

| **Angle (2-Theta)** (each time ±0.2) | **Relative intensity (%)** |
|---|---|
| 7.6 | 12 |
| 7.9 | 60 |
| 14.0 | 33 |
| 14.7 | 11 |
| 15.2 | 78 |
| 15.8 | 41 |
| 16.1 | 16 |
| 16.9 | 28 |
| 18.5 | 23 |
| 19.7 | 17 |
| 19.9 | 26 |
| 20.3 | 43 |
| 21.6 | 13 |
| 22.0 | 25 |
| 22.3 | 20 |
| 23.0 | 27 |
| 23.7 | 25 |
| 24.0 | 33 |
| 24.7 | 45 |
| 25.2 | 100 |

According to one embodiment, the co-crystal of 8-Chloro-N-(4-(trifluoromethoxy)phenyl)quinolin-2-amine :Gentisic acid of the present invention presents a powder X-ray diffractogram displaying at least one peak expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least two peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least three peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least four peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least five peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least six peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least seven peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least eight peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least nine peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least ten peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least eleven peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least twelve peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least thirteen peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least fourteen peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention presents a powder X-ray diffractogram displaying at least fifteen peaks expressed as degree 2-Theta angle selected from 7.9; 14.0; 15.2; 25.2; 15.8; 16.9; 18.5; 19.9; 20.3; 23.0; 24.7; 7.6; 14.7; 16.1; 19.7; 21.6; 22.0; 22.3; 23.7; and 24.0 (each time ±0.2).

According to another embodiment, the co-crystal of 8-Chloro-N-(4-(trifluoromethoxy)phenyl)quinolin-2-amine :Gentisic acid of the present invention has an XRPD substantially similar to that depicted in figure 2.

A characteristic X-ray powder diffractogram of a co-crystal of 8-chloro-N-(4-(trifluoromethoxy)phenyl)quinolin-2-amine : Malonic acid can be given in figure 3 and its characteristic signals are summarized in the following table 6 :

**Table 6**

| **Angle (2-Theta)** (each time ±0.2) | **Relative intensity (%)** |
|---|---|
| 9.5 | 26 |
| 12.2 | 34 |
| 15.8 | 38 |
| 16.8 | 7 |
| 17.3 | 42 |
| 17.8 | 7 |
| 19.0 | 22 |
| 19.7 | 47 |
| 20.9 | 6 |
| 21.4 | 27 |
| 22.8 | 65 |
| 23.8 | 11 |
| 24.6 | 76 |
| 24.8 | 100 |
| 25.6 | 54 |
| 26.8 | 18 |
| 27.6 | 31 |
| 28.0 | 11 |
| 29.6 | 13 |
| 29.9 | 25 |

According to one embodiment, the co-crystal of 8-Chloro-N-(4-(trifluoromethoxy)phenyl)quinolin-2-amine : Malonic acid of the present invention presents a powder X-ray diffractogram displaying at least one peak expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least two peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least three peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least four peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least five peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least six peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least seven peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least eight peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least nine peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least ten peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least eleven peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least twelve peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least thirteen peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least fourteen peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention presents a powder X-ray diffractogram displaying at least fifteen peaks expressed as degree 2-Theta angle selected from 9.5; 12.2; 15.8; 17.3; 19.7; 22.8; 24.8; 25.6; 19.0; 21.4; 24.6; 26.8; 27.6; 29.9; 16.8; 17.8; 20.9; 23.8; 28.0; and 29.6 (each time ±0.2).

According to another embodiment, the co-crystal of 8-Chloro-N-(4-(trifluoromethoxy)phenyl)quinolin-2-amine : Malonic acid of the present invention has an XRPD substantially similar to that depicted in figure 3.

A characteristic X-ray powder diffractogram of a co-crystal of 8-chloro-N-(4-(trifluoromethoxy)phenyl)quinolin-2-amine : 4, 4'-Bipyridine can be given in figure 4 and its characteristic signals are summarized in the following table 7:

**Table 7**

| **Angle (2-Theta)** (each time ±0.2) | **Relative intensity (%)** |
|---|---|
| 8.5 | 5 |
| 12.0 | 16 |
| 13.0 | 7 |
| 15.7 | 7 |
| 16.0 | 12 |
| 16.7 | 9 |
| 17.0 | 30 |
| 17.8 | 16 |
| 19.2 | 40 |
| 20.3 | 14 |
| 20.9 | 11 |
| 21.2 | 100 |
| 22.0 | 8 |
| 22.5 | 21 |
| 22.7 | 21 |
| 23.1 | 10 |
| 23.6 | 12 |
| 24.3 | 36 |
| 24.7 | 10 |

According to one embodiment, the co-crystal of 8-chloro-N-(4-(trifluoromethoxy)phenyl)quinolin-2-amine : 4, 4'-Bipyridine of the present invention presents a powder X-ray diffractogram displaying at least one peak expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least two peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least three peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least four peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least five peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least six peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least seven peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least eight peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least nine peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least ten peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least eleven peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least twelve peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least thirteen peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least fourteen peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention presents a powder X-ray diffractogram displaying at least fifteen peaks expressed as degree 2-Theta angle selected from 12.0; 19.2; 21.2; 24.3; 16.0; 17.0; 17.8; 20.3; 22.5; 22.7; 8.5; 13.0; 15.7; 16.7; 20.9; 22.0; 23.1; 23.6 and 24.7 (each time ±0.2).

According to another embodiment, the co-crystal of 8-chloro-N-(4-(trifluoromethoxy)phenyl)quinolin-2-amine : 4, 4'-Bipyridine of the present invention has an XRPD substantially similar to that depicted in figure 4.

### METHOD OF PREPARING CO-CRYSTALS OF ABX464

According to another aspect, the present invention further relates to a method of preparing a co-crystal of 8-chloro-N-(4-(trifluoromethoxy)phenyl)quinolin-2-amine as defined in the present invention comprising the following steps of :
a) dissolving ABX464 in a solvent or in a mixture of solvents;
b) adding to the thus obtained mixture of step a) a co-former which may be itself already dissolved in a solvent or in a mixture of solvents and which is selected from L-proline, gentisic acid, malonic acid and 4,4'-bipyridine so as to obtain a ABX464 : co-former in molar ratio comprised between 3:1 and 1:2, particularly between 5:2 and 1:2; more particularly between 2:1 and 1:2, and still more particularly is of 2:1 or 1:1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent or a mixture of solvents,
e) applying a temperature program;
f) optionally filtrating; and
g) then optionally drying at a temperature comprised between room temperature and 60°C in order to obtain the desired co-crystal of ABX464.

According to one embodiment, the solvent(s) used in step a), step b) and step d) is(are) any solvent conventionally used in crystallization step, particularly is(are) organic solvents, more particularly is(are) selected from a C₁-C₆ aliphatic alcohol, methyl ethyl ketone (also named butanone or MEK), cyclohexane, alkane such as heptane, methylene chloride, chloroform, formic acid, DMSO, 1-methyl-2-pyrrolidone, acetone, acetonitrile, tetrahydrofuran (THF), diethyl ether, dioxane, toluene, ethyl acetate, optionally in admixture with water, and mixtures thereof, still more particularly selected from a C₁-C₆ aliphatic alcohol, a mixture of H₂O/ C₁-C₆ aliphatic alcohol, acetonitrile, and mixtures thereof, even more particularly selected from methanol, ethanol, isopropanol, H₂O/methanol, H₂O/ethanol, acetonitrile, and mixtures thereof.

The skilled person would know how to determine the more appropriate solvent(s) in each step a), b) and d) so as to obtain the desired co-crystal.

According to one embodiment, the solvent(s) used in step a), step b) and/or step d) is(are) the same.

According to another embodiment, the solvent(s) used in step a), step b) and/or step d) is(are) different.

According to one embodiment, the evaporating step c) is carried out under an inert gas such as N₂.

According to one embodiment the step e) relative to the temperature program includes a heating phase at reflux to a temperature comprised between room temperature and the boiling point of the solvent(s), particularly between room temperature and 60°C.

According to one embodiment the step e) relative to the temperature program includes a cooling phase at reflux to a temperature comprised between 0°C and 60°C, particularly between 5°C and 40°C, more particularly between room temperature and 40°C at a rate comprised between 30°C/min and 0.05°C/min, particularly between 10°C/min and 0.05°C/min, more particularly between 5°C/min and 0.05°C/min.

According to one embodiment, the step e) relative to the temperature program includes i) a heating at reflux to a temperature comprised between room temperature and the boiling point of the solvent(s), particularly between room temperature and 60°C and / or ii) a cooling at reflux to a temperature comprised between 0°C and 60°C, particularly between 5°C and 40°C, more particularly between room temperature and 40°C at a rate comprised between 30°C/min and 0.05°C/min, particularly between 10°C/min and 0.05°C/min, more particularly between 5°C/min and 0.05°C/min.

According to a particular embodiment, the step e) relative to the temperature program includes i) a heating at reflux to a temperature comprised between room temperature and 60°C and ii) a cooling at reflux to a temperature comprised between 5°C and 40°C, at a rate comprised between 30°C/min and 0.05°C/min, particularly between 10°C/min and 0.05°C/min, more particularly between 5°C/min and 0.05°C/min.

According to one embodiment, the step f) regarding the filtration is carried out using conventional glass fibers, conventional cellulosic filter papers, PTFE (polytetrafluoroethylene), or PVDF (polyvinylidene fluoride), particularly cellulosic filter paper with 0.45µm or 0.2µm filtration mesh.

According to one embodiment, the step g) regarding the drying is carried out under vacuum at a temperature comprised between 30°C and 60°C, particularly at 40°C.

According to one embodiment, the step g) regarding the drying is carried out under ambient atmosphere at a temperature comprised between 30°C and 60°C, particularly at 40°C.

According to a particular embodiment, the method of preparing a co-crystal of 8-chloro-N-(4-(trifluoromethoxy)phenyl)quinolin-2-amine as defined in the present invention comprises the following steps of :
a) dissolving ABX464 in a solvent or in a mixture of solvents;
b) adding to the thus obtained mixture of step a) a co-former which may be itself already dissolved in a solvent or in a mixture of solvents and which is selected from L-proline, gentisic acid, malonic acid and 4,4'-bipyridine so as to obtain a ABX464 : co-former in molar ratio comprised between 3:1 and 1:2, particularly between 5:2 and 1:2; more particularly between 2:1 and 1:2, and still more particularly is of 2:1 or 1:1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent or a mixture of solvents,
e) applying a temperature program including i) a heating at reflux to a temperature comprised between room temperature and 60°C and / or ii) a cooling at reflux to a temperature comprised between 5°C and 40°C, at a rate comprised between 30°C/min and 0.05°C/min, particularly between 10°C/min and 0.05°C/min, more particularly between 5°C/min and 0.05°C/min;
f) optionally filtrating by using conventional glass fibers, conventional cellulosic filter papers, PTFE (polytetrafluoroethylene), or PVDF (polyvinylidene fluoride), particularly cellulosic filter paper with 0.45µm or 0.2µm filtration mesh; and
g) then optionally drying under vacuum at a temperature comprised between 30°C and 60°C, particularly at 40°C or under ambient atmosphere at a temperature comprised between 30°C and 60°C, particularly at 40°C in order to obtain the desired co-crystal of ABX464.

According to another particular embodiment, the method of preparing a co-crystal of 8-chloro-N-(4-(trifluoromethoxy)phenyl)quinolin-2-amine as defined in the present invention comprises the following steps of :
a) dissolving ABX464 in methanol, ethanol, isopropanol, H₂O/methanol, H₂O/ethanol, acetonitrile, or mixtures thereof;
b) adding to the thus obtained mixture of step a) a co-former which may be itself already dissolved in methanol, ethanol, isopropanol, H₂O/methanol, H₂O/ethanol, acetonitrile, or mixtures thereof and which is selected from L-proline, gentisic acid, malonic acid and 4,4'-bipyridine so as to obtain a ABX464 : co-former in molar ratio comprised between 3:1 and 1:2, particularly between 5:2 and 1:2; more particularly between 2:1 and 1:2, and still more particularly is of 2:1 or 1:1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent which is selected from methanol, ethanol, isopropanol, H₂O/methanol, H₂O/ethanol, acetonitrile, and mixtures thereof,
e) applying a temperature program;
f) optionally filtrating; and
g) then optionally drying at a temperature comprised between room temperature and 60°C in order to obtain the desired co-crystal of ABX464.

According to another particular embodiment, the method of preparing a co-crystal of 8-chloro-N-(4-(trifluoromethoxy)phenyl)quinolin-2-amine as defined in the present invention comprises the following steps of :
a) dissolving ABX464 in methanol, ethanol, isopropanol, H₂O/methanol, H₂O/ethanol, acetonitrile, or mixtures thereof;
b) adding to the thus obtained mixture of step a) a co-former which may be itself already dissolved in methanol, ethanol, isopropanol, H₂O/methanol, H₂O/ethanol, acetonitrile, or mixtures thereof and which is selected from L-proline, gentisic acid, malonic acid and 4,4'-bipyridine so as to obtain a ABX464 : co-former in molar ratio comprised between 3:1 and 1:2, particularly between 5:2 and 1:2; more particularly between 2:1 and 1:2, and still more particularly is of 2:1 or 1:1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent which is selected from methanol, ethanol, isopropanol, H₂O/methanol, H₂O/ethanol, acetonitrile, and mixtures thereof,
e) applying a temperature program including i) a heating at reflux to a temperature comprised between room temperature and 60°C and / or ii) a cooling at reflux to a temperature comprised between 5°C and 40°C, at a rate comprised between 30°C/min and 0.05°C/min, particularly between 10°C/min and 0.05°C/min, more particularly between 5°C/min and 0.05°C/min;
f) optionally filtrating by using conventional glass fibers, conventional cellulosic filter papers, PTFE (polytetrafluoroethylene), or PVDF (polyvinylidene fluoride), particularly cellulosic filter paper with 0.45µm or 0.2µm filtration mesh; and
g) then optionally drying under vacuum at a temperature comprised between 30°C and 60°C, particularly at 40°C or under ambient atmosphere at a temperature comprised between 30°C and 60°C, particularly at 40°C in order to obtain the desired co-crystal of ABX464.

According to another aspect, the present invention further relates to a method of preparing a co-crystal of 8-chloro-N-(4-(trifluoromethoxy)phenyl)quinolin-2-amine as defined in the present invention comprising the steps of :
- a') physically mixing 8-chloro-N-(4-(trifluoromethoxy)phenyl)quinolin-2-amine with a co-former chosen among L-Proline, Gentisic acid, Malonic acid and 4, 4'-Bipyridine at a molar ratio comprised between 2:1 and 1:2, particularly at a 1:1 molar ratio, in a suitable solvent or mixtures of solvents; and
- b') grinding of the physical mixture thus obtained from step a), in presence of one drop of solvent or of mixtures of solvents to obtain the co-crystal.

The skilled person would know how to determine the more appropriate solvent(s) in each step a') and b') so as to obtain the desired co-crystal.

According to one embodiment, the solvent(s) used in step a') and step b') is(are) any solvent conventionally used in crystallization step, particularly is(are) organic solvents, more particularly is(are) selected from a C₁-C₆ aliphatic alcohol, methyl ethyl ketone (also named butanone or MEK), cyclohexane, alkane such as heptane, methylene chloride, chloroform, formic acid, DMSO, 1-methyl-2-pyrrolidone, acetone, acetonitrile, tetrahydrofuran (THF), diethyl ether, dioxane, toluene, ethyl acetate, optionally in admixture with water, and mixtures thereof, still more particularly selected from a C₁-C₆ aliphatic alcohol, a mixture of H₂O/ C₁-C₆ aliphatic alcohol, acetonitrile, and mixtures thereof, even more particularly selected from methanol, ethanol, isopropanol, H₂O/methanol, H₂O/ethanol, acetonitrile, and mixtures thereof.

In some embodiments, the grinding is carried out by using Retsch MM200 instrument for 45 min milling at 20 Hz.

According to one embodiment, the grinding is carried out by using non-oxidizable balls.

According to one embodiment, the grinding is carried out by using horizontal movements, particularly horizontal movements having a frequency ranging from 20 to 30 Hz.

Advantageously, the horizontal movements are applied for a period which may range from 15 minutes to 3 hours, particularly 45 min.

According to a particular embodiment, the present invention further relates to a method of preparing a co-crystal of 8-chloro-N-(4-(trifluoromethoxy)phenyl)quinolin-2-amine as defined in the present invention comprising the steps of :
- a') physically mixing 8-chloro-N-(4-(trifluoromethoxy)phenyl)quinolin-2-amine with a co-former chosen among L-Proline, Gentisic acid, Malonic acid and 4, 4'-Bipyridine at a molar ratio comprised between 2:1 and 1:2, particularly at a 1:1 molar ratio, in a suitable solvent which is selected from methanol, ethanol, isopropanol, H₂O/methanol, H₂O/ethanol, acetonitrile, and mixtures thereof; and
- b') grinding of the physical mixture thus obtained from step a), in presence of one drop of solvent which is selected from methanol, ethanol, isopropanol, H₂O/methanol, H₂O/ethanol, acetonitrile, and mixtures thereof to obtain the co-crystal.

It is understood that for the preparation of a co-crystal of ABX464, 8-chloro-N-(4-(trifluoromethoxy)phenyl)quinolin-2-amine can be previously obtained either from a process as the one described in WO2010/143169 or from any other appropriate process.

### CO-CRYSTAL OF ABX 464, PHARMACEUTICALLY ACCEPTABLE SALTS OF ABX 464 AND PHARMACEUTICAL COMPOSITIONS IN ACCORDANCE WITH THE INVENTION FOR USE AS MEDICINES

According to another aspect, the present invention further relates to a pharmaceutical composition comprising as (an) active ingredient(s) at least one co-crystal of 8-chloro-N-(4-(trifluoromethoxy)phenyl)quinolin-2-amine as defined in the present invention and / or at least one pharmaceutically acceptable salts of 8-chloro-N-(4-(trifluoromethoxy)phenyl)quinolin-2-amine including a solvate and/or a hydrate thereof as defined in the present invention, and at least one pharmaceutically acceptable excipient.

According to another aspect, the present invention further relates to a pharmaceutical composition, wherein the pharmaceutical composition comprises at least one of the co-crystals as defined in the present invention and / or at least one of the pharmaceutically acceptable salts of 8-chloro-N-(4-(trifluoromethoxy)phenyl)quinolin-2-amine including a solvate and/or a hydrate thereof as defined in the present invention, as the sole pharmaceutically active ingredient(s).

Among the pharmaceutical compositions according to the invention there may be mentioned, more especially, those that are suitable for oral, parenteral (intravenous or subcutaneous) or nasal administration, tablets or dragees, granules, sublingual tablets, capsules, lozenges, suppositories, creams, ointments, dermal gels, injectable preparations, drinkable suspensions and chewing gums.

The useful dosage can be varied according to the nature and severity of the disorder, the administration route and the age and weight of the patient.

According to one embodiment, a pharmaceutical composition in accordance with the invention is such that a dose of from 1 mg to 1 g per day, particularly of from 10 mg to 150 mg per day of active ingredient ABX464 is administered to a subject in need thereof in one or more doses per day.

The excipients can be any conventional used excipients including intragranular excipients, and/or an extragranular excipients.

The excipients may be selected from fillers, glidants, binders, antioxidants, disintegrants, lubricants, surfactants, and mixtures thereof.

Fillers which are useable in accordance with the invention include, but are not limited to, lactose (anhydrous), lactose monohydrate, spray-dried lactose; compressible sugar, dextrose, dextrates; starches (including starches from any source, such as corn, potato, rice, wheat, which can be fully pregelatinized and partially gelatinized); cellulose; microcrystalline cellulose ; inorganic salts such as calcium phosphate, tribasic calcium and calcium sulfate; and polyols such as mannitol, sorbitol and xylitol.

In some embodiments, the filler may be in an amount of 10% to 85% by weight based on the total weight of the composition.

Lubricants which are useable according to the invention include, but are not limited to, magnesium stearate, calcium stearate, zinc stearate, stearic acid, sodium stearyl fumarate, hydrogenated vegetable oils, mineral oil, polyethylene glycols, talc, glyceryl behenate, glyceryl monostearate, glyceryl palmitostearate, leucine, and magnesium lauryl sulfate.

In some embodiments, the lubricant may be in an amount of 0.3% to 2% by weight based on the total weight of the composition.

Disintegrants which are useable in accordance with the invention include, but are not limited to, croscarmellose sodium, sodium starch glycolate, starches (including starches from any source, such as corn, potato, rice, wheat, fully pregelatinized and partially gelatinized), crospovidone, alginates such as calcium alginate and sodium alginate, alginic acid, and magnesium aluminum silicate.

In some embodiments, the disintegrant may be in an amount of 30% to 60% by weight based on the total weight of composition.

The surfactants employable as an additive in the present invention include, but are not limited to, sodium lauryl sulfate, tocopherol, lecithin, lauryl sulfate, Vitamin E, egg yolk phosphatides, docusate sodium, Capryol, Labrafil, Labrasol, Lauroglycol, Solutol (Macrogol-15 hydroxystearate), and mixtures thereof.

In some embodiments, the surfactant may be in an amount of 1% to 3% by weight based on the total weight of composition.

In some embodiments, the glidant may be in an amount of 0.3% to 2% by weight based on the total weight of composition.

In some embodiments, the binder may be in an amount of 5% to 20% by weight based on the total weight of composition.

According to another aspect, the present invention also relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, for use as a medicine.

According to another aspect, the present invention also relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, for use in the prevention and /or treatment of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia.

According to another aspect, the present invention also relates to the use of a co-crystal of 8-chloro-N-(4-(trifluoromethoxy)phenyl)quinolin-2-amine as defined in the present invention, of a pharmaceutically acceptable salt of 8-chloro-N-(4-(trifluoromethoxy)phenyl)quinolin-2-amine including a solvate and/or a hydrate thereof as defined in the present invention, or of a pharmaceutical composition as defined in the present invention for the manufacture of a medicament.

According to another aspect, the present invention also relates to the use of a co-crystal of 8-chloro-N-(4-(trifluoromethoxy)phenyl)quinolin-2-amine as defined in the present invention, of a pharmaceutically acceptable salt of 8-chloro-N-(4-(trifluoromethoxy)phenyl)quinolin-2-amine including a solvate and/or a hydrate thereof as defined in the present invention, or of a pharmaceutical composition as defined in the present invention for the manufacture of a medicament for preventing and /or treating of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia.

According to another aspect, the present invention also relates to a therapeutic method of treating and/or preventing of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia comprising administering to a patient in need thereof a composition comprising a co-crystal of 8-chloro-N-(4-(trifluoromethoxy)phenyl)quinolin-2-amine as defined in the present invention and / or a pharmaceutically acceptable salt of 8-chloro-N-(4-(trifluoromethoxy)phenyl)quinolin-2-amine including a solvate and/or a hydrate thereof as defined in the present invention.

According to another aspect, the present invention also relates to a therapeutic method of treating and/or preventing of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia comprising administering to a patient in need thereof a therapeutically effective amount of a co-crystal of 8-chloro-N-(4-(trifluoromethoxy)phenyl)quinolin-2-amine as defined in the present invention and / or a pharmaceutically acceptable salt of 8-chloro-N-(4-(trifluoromethoxy)phenyl)quinolin-2-amine including a solvate and/or a hydrate thereof as defined in the present invention.

A method for administering a co-crystal of ABX464 to a subject in need thereof is provided, comprising:
- providing an oral pharmaceutical composition comprising: a co-crystal of ABX464 and / or a pharmaceutically acceptable salt of 8-chloro-N-(4-(trifluoromethoxy)phenyl)quinolin-2-amine including a solvate and/or a hydrate thereof as defined in the present invention, and at least one pharmaceutically acceptable excipient; and
- orally administering the pharmaceutical composition in a therapeutically effective amount to a subject in need thereof.

A co-crystal of ABX464 as defined in the present invention can be administered alone or in combination with other therapeutic agents which can act synergistically with said co-crystal of ABX464 as defined in the present invention. For example, another therapeutic agent can be a pharmaceutically acceptable salt of 8-chloro-N-(4-(trifluoromethoxy)phenyl)quinolin-2-amine including a solvate and/or a hydrate thereof as defined in the present invention.

By analogy, a pharmaceutically acceptable salt of 8-chloro-N-(4-(trifluoromethoxy)phenyl)quinolin-2-amine including a solvate and/or a hydrate thereof as defined in the present invention can be administered alone or in combination with other therapeutic agents which can act synergistically with said pharmaceutically acceptable salt of ABX464 including a solvate and/or a hydrate thereof as defined in the present invention. For example, another therapeutic agent can be a co-crystal of ABX464 as defined in the present invention.

### Inflammatory diseases

Thus, the invention also relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, for use in the treatment and/or prevention of an inflammatory disease.

According to the invention, an « *inflammation* » is a protective response by the immune system to tissue damage and infection. However, the inflammatory response, in some circumstances, can damage the body. In the acute phase, inflammation is characterized by pain, heat, redness, swelling and loss of function. Inflammation can result from infection, irritation, or injury.

Thus, an « *inflammatory disease* » refers to a group of diseases and/or disorders that are caused by an excessive or dysregulated inflammation.

In a non-limitative manner, inflammatory diseases include: an inflammatory disease associated with an autoimmune disease, a central nervous system (CNS) inflammatory disease, a joint inflammation disease, an inflammatory digestive tract disease, inflammatory skin and other inflammatory diseases related to epithelial cells such as bronchitis, inflammation associated with cancer, such as colon carcinoma, inflammation associated with irritation, and inflammation associated with injury.

According to the present invention, the inflammatory disease, disorder or condition is selected from:
(a) an inflammatory disease, disorder, or condition in the pancreas selected from diabetes type-1, diabetes type-2, acute and chronic pancreatitis;
(b) an inflammatory disease, disorder, or condition in the kidney selected from glomerulosclerosis, glomerulonephritis, nephritis, acute kidney injury, Berger's disease, Goodpasture's syndrome, Wegener's granulomatosis and kidney transplant acute or chronic rejection;
(c) an inflammatory disease, disorder, or condition in the liver selected from nonalcoholic steatohepatitis (NASH), non-alcoholic fatty liver disease (NAFLD), cholestatic liver disease, sclerosing cholangitis and liver transplant acute or chronic rejection;
(d) an inflammatory disease, disorder, or condition in the lung or heart selected from chronic obstructive pulmonary disease (COPD), **asthma,** pulmonary fibrosis, pulmonary arterial hypertension, sarcoidosis, myocarditis, pericarditis and lung or heart transplant acute or chronic rejection;
(e) an inflammatory disease, disorder, or condition in the skin selected from contact dermatitits, atopic dermatitis, urticaria, chronic dermatitis, psoriasis, eczema, alopecia areata, erythema multiforma, dermatitis herpetiformis, scleroderma, vitiligo, hypersensitivity angiitis, urticaria, bullous pemphigoid, pemphigus vulgaris, pemphigus foliaceus, paraneoplastic pemphigus, epidermolysis bullosa acquisita, acnea, keloid scar, and other inflammatory or allergic conditions of the skin;
(f) an inflammatory disease, disorder, or condition in the vessel/blood selected from Behcet's disease, vasculitis, sepsis, tumor angiogenesis, atherosclerosis, proliferative vascular disease and restenosis;
(g) an inflammatory disease, disorder, or condition in the eye selected from conjunctivitis, scleritis, episcleritis, panuveitis, choroiditis, chorioretinitis, neuroretinitis, uveitis, orbital inflammatory disease, and optical neuritis;
(h) an inflammatory disease, disorder, or condition in the central or peripheral nervous system selected from non-viral and viral encephalitis and meningitis, depression, neuropathic pain, chronic pain, traumatic brain injury, including stroke, Alzheimer disease, Parkinson disease, Myelitis, Charcot-Marie-Tooth disease type 1 (including CMT1A and CMT1B), Multiple Sclerosis, Amyotrophic lateral sclerosis (ALS), Creutzfeldt-Jakob disease, demyelinating polyneuropathy and peripheral neuropathy;
(i) an autoimmune disease, disorder, or condition selected from Lupus, including in the skin and kidney, Guillain-Barre syndrome, Myasthenia gravis, Hashimoto's thyroiditis, idiopathic purpura, aplastic anemia, Graves disease, and Myocarditis;
(j) an inflammatory disease, disorder, or condition in the intestine selected from intestinal failure, Ulcerative colitis (UC) and Crohn's disease,
(k) an inflammatory disease, disorder, or condition in the reproductive system selected from endometriosis, uterine fibroma, prostate dysplasia or growth, and cervix dysplasia; and
(l) an inflammatory disease, disorder, or condition in the bone and/or joints selected from rheumatoid arthritis, juvenile idiopathic arthritis, psoriatic arthritis, periodontitis, and hand, foot, ankle, knee, hip, shoulder, elbow or spine arthritis and/or demineralization.

In a particular embodiment, the inflammatory disease can be selected in the list consisting of: an inflammatory disease associated with an autoimmune disease, a central nervous system (CNS) inflammatory disease, a joint inflammation disease, an inflammatory digestive tract disease, inflammatory skin and other inflammatory diseases related to epithelial cells, inflammation associated with cancer, inflammation associated with irritation, and inflammation associated with injury.

In particular, an inflammatory disease is selected in the list consisting of: Inflammatory Bowel Disease, Rheumatoid Arthritis, Crohn's disease, Ulcerative Colitis, Multiple Sclerosis, Alzheimer's disease, Parkinson, osteoarthritis, atherosclerosis, ankylosing spondylitis, psoriasis, dermatitis, Sjogren's syndrom, bronchitis, asthma, pulmonary arterial hypertension, NASH and inflammation associated with colon carcinoma.

More particularly, an inflammatory disease is selected in the list consisting of: Inflammatory Bowel Disease, Rheumatoid Arthritis, Crohn's disease, Ulcerative Colitis, Multiple Sclerosis, osteoarthritis, ankylosing spondylitis, psoriasis, Sjogren's syndrom, bronchitis, pulmonary arterial hypertension, NASH and inflammation associated with colon carcinoma.

More particularly, an inflammatory disease is selected in the list consisting of: Inflammatory Bowel Disease, Rheumatoid Arthritis, Crohn's disease, Ulcerative Colitis, Multiple Sclerosis, osteoarthritis, ankylosing spondylitis, pulmonary arterial hypertension, NASH and psoriasis.

Preferably, an inflammatory disease according to the invention includes: Inflammatory Bowel Disease, Crohn's disease, Ulcerative Colitis, Rheumatoid Arthritis, pulmonary arterial hypertension, NASH and Multiple Sclerosis.

Even more preferably, an inflammatory disease according to the invention includes: Inflammatory Bowel Disease, Rheumatoid Arthritis, pulmonary arterial hypertension, NASH and Multiple Sclerosis.

An inflammatory disease may also encompass Alzheimer's disease, Parkinson, asthma, atherosclerosis and dermatitis.

As dermatitis, eczema may be cited.

In view of the above, the invention relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use in the treatment and/or prevention of an inflammatory disease, which encompasses inflammation as such, and inflammation associated with an inflammatory disease.

Thus, the invention also relates to the use of a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for treating and/or preventing an inflammatory disease, which encompasses inflammation as such, and inflammation associated with an inflammatory disease.

The invention also relates to the use of a co-crystal of 8-chloro-N-(4-(trifluoromethoxy)phenyl)quinolin-2-amine as defined in the present invention, or a pharmaceutically acceptable salt of 8-chloro-N-(4-(trifluoromethoxy)phenyl)quinolin-2-amine including a solvate and/or a hydrate thereof as defined in the present invention, for the preparation of a composition, such as a medicament, for treating and/or preventing inflammation, which encompasses inflammation as such, and inflammation associated with an inflammatory disease.

The invention also relates to a method for treating and/or preventing an inflammatory disease, which includes inflammation as such, and inflammation associated with said inflammatory disease, and which comprises a step of administering a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention to a patient in need thereof.

In some embodiments, a method of the present invention or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, for treating an inflammatory disease, disorder or condition further comprises measuring and/or monitoring a presence and/or level of a biomarker in a patient, for example in a blood, plasma, tissue, saliva, and/or serum sample. In some embodiments, a biomarker measured and/or monitored in a method of the present invention is miR-124.

In some embodiments, a method of the present invention or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, for treating an inflammatory disease, disorder or condition further comprises measuring and/or monitoring a presence and/or expression level of miR-124 in a patient, for example in a blood, plasma, tissue, saliva, and/or serum sample, prior to administering a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention as described herein.

In some embodiments, a method of the present invention or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, for treating an inflammatory disease, disorder or condition further comprises measuring and/or monitoring a presence and/or expression level of miR-124 in a patient during the course of a treatment with a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention thereof as described herein.

In some embodiments, a method of the present invention for treating an inflammatory disease, disorder or condition, or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, further comprises selecting a patient for a treatment with a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention thereof as described herein, by measuring and/or monitoring a presence and/or expression level of miR-124 in the patient.

A provided co-crystal of 8-chloro-N-(4-(trifluoromethoxy)phenyl)quinolin-2-amine as defined in the present invention, or a provided pharmaceutically acceptable salt of 8-chloro-N-(4-(trifluoromethoxy)phenyl)quinolin-2-amine including a solvate and/or a hydrate thereof as defined in the present invention can be administered alone or in combination with one or more other therapeutic compounds, possible combination therapy taking the form of fixed combinations or the administration of a compound of the invention and one or more other therapeutic compounds being staggered or given independently of one another, or the combined administration of fixed combinations and one or more other therapeutic compounds. A compound of the current invention can besides or in addition be administered especially for tumor therapy in combination with chemotherapy, radiotherapy, immunotherapy, phototherapy, surgical intervention, or a combination of these. Long-term therapy is equally possible as is adjuvant therapy in the context of other treatment strategies, as described above. Other possible treatments are therapy to maintain the patient's status after tumor regression, or even chemopreventive therapy, for example in patients at risk.

Those additional agents may be administered separately from a provided co-crystal of 8-chloro-N-(4-(trifluoromethoxy)phenyl)quinolin-2-amine as defined in the present invention, or from a provided pharmaceutically acceptable salt of 8-chloro-N-(4-(trifluoromethoxy)phenyl)quinolin-2-amine including a solvate and/or a hydrate thereof as defined in the present invention, as part of a multiple dosage regimen. Alternatively, those agents may be part of a single dosage form, mixed together with a provided co-crystal of 8-chloro-N-(4-(trifluoromethoxy)phenyl)quinolin-2-amine as defined in the present invention, or with a provided pharmaceutically acceptable salt of 8-chloro-N-(4-(trifluoromethoxy)phenyl)quinolin-2-amine including a solvate and/or a hydrate thereof as defined in the present invention in a single composition. If administered as part of a multiple dosage regime, the two active agents may be submitted simultaneously, sequentially or within a period of time from one another normally within five hours from one another.

As used herein, the term "combination," "combined," and related terms refers to the simultaneous or sequential administration of therapeutic agents in accordance with this invention. For example, a provided co-crystal of 8-chloro-N-(4-(trifluoromethoxy)phenyl)quinolin-2-amine as defined in the present invention, or a provided pharmaceutically acceptable salt of 8-chloro-N-(4-(trifluoromethoxy)phenyl)quinolin-2-amine including a solvate and/or a hydrate thereof as defined in the present invention may be administered with another therapeutic agent simultaneously or sequentially in separate unit dosage forms or together in a single unit dosage form. Accordingly, the present invention provides a single unit dosage form comprising a provided co-crystal of 8-chloro-N-(4-(trifluoromethoxy)phenyl)quinolin-2-amine as defined in the present invention, or a provided pharmaceutically acceptable salt of 8-chloro-N-(4-(trifluoromethoxy)phenyl)quinolin-2-amine including a solvate and/or a hydrate thereof as defined in the present invention, an additional therapeutic agent, and a pharmaceutically acceptable carrier, adjuvant, or vehicle.

In some embodiments, the co-crystal of 8-chloro-N-(4-(trifluoromethoxy)phenyl)quinolin-2-amine as defined in the present invention, or the pharmaceutically acceptable salt of 8-chloro-N-(4-(trifluoromethoxy)phenyl)quinolin-2-amine including a solvate and/or a hydrate thereof as defined in the present invention may be administered with one or more additional therapeutic agents. Such additional therapeutic agents may be small molecules or recombinant biologic agents and include, for example, acetaminophen, non-steroidal anti-inflammatory drugs (NSAIDS) such as aspirin, ibuprofen, naproxen, etodolac (Lodine®) and celecoxib, colchicine (Colcrys®), corticosteroids such as prednisone, prednisolone, methylprednisolone, hydrocortisone, and the like, probenecid, allopurinol, febuxostat (Uloric®), sulfasalazine (Azulfidine®), antimalarials such as hydroxychloroquine (Plaquenil®) and chloroquine (Aralen®), methotrexate (Rheumatrex®), gold salts such as gold thioglucose (Solganal®), gold thiomalate (Myochrysine®) and auranofin (Ridaura®), D-penicillamine (Depen® or Cuprimine®), azathioprine (Imuran®), cyclophosphamide (Cytoxan®), chlorambucil (Leukeran®), cyclosporine (Sandimmune®, Neoral®), tacrolimus, sirolimus, mycophenolate, leflunomide (Arava®) and "anti-TNF" agents such as etanercept (Enbrel®), infliximab (Remicade®), golimumab (Simponi®), certolizumab pegol (Cimzia®) and adalimumab (Humira®), "anti-IL-1" agents such as anakinra (Kineret®) and rilonacept (Arcalyst®), anti-T cell antibodies such as Thymoglobulin, IV Immunoglobulins (IVIg), canakinumab (Ilaris®), anti-Jak inhibitors such as tofacitinib, antibodies such as rituximab (Rituxan®), "anti-T-cell" agents such as abatacept (Orencia®), "anti-IL-6" agents such as tocilizumab (Actemra®), diclofenac, cortisone, hyaluronic acid (Synvisc® or Hyalgan®), monoclonal antibodies such as tanezumab, anticoagulants such as heparin (Calcinparine® or Liquaemin®) and warfarin (Coumadin®), antidiarrheals such as diphenoxylate (Lomotil®) and loperamide (Imodium®), bile acid binding agents such as cholestyramine, alosetron (Lotronex®), lubiprostone (Amitiza®), laxatives such as Milk of Magnesia, polyethylene glycol (MiraLax®), Dulcolax®, Correctol® and Senokot®, anticholinergics or antispasmodics such as dicyclomine (Bentyl®), Singulair®, beta-2 agonists such as albuterol (Ventolin® HFA, Proventil® HFA), levalbuterol (Xopenex®), metaproterenol (Alupent®), pirbuterol acetate (Maxair®), terbutaline sulfate (Brethaire®), salmeterol xinafoate (Serevent®) and formoterol (Foradil®), anticholinergic agents such as ipratropium bromide (Atrovent®) and tiotropium (Spiriva®), inhaled corticosteroids such as beclomethasone dipropionate (Beclovent®, Qvar®, and Vanceril®), triamcinolone acetonide (Azmacort®), mometasone (Asthmanex®), budesonide (Pulmocort®), and flunisolide (Aerobid®), Afviar®, Symbicort®, Dulera®, cromolyn sodium (Intal®), methylxanthines such as theophylline (Theo-Dur®, Theolair®, Slo-bid®, Uniphyl®, Theo-24®) and aminophylline, IgE antibodies such as omalizumab (Xolair®), nucleoside reverse transcriptase inhibitors such as zidovudine (Retrovir®), abacavir (Ziagen®), abacavir/lamivudine (Epzicom®), abacavir/lamivudine/zidovudine (Trizivir®), didanosine (Videx®), emtricitabine (Emtriva®), lamivudine (Epivir®), lamivudine/zidovudine (Combivir®), stavudine (Zerit®), and zalcitabine (Hivid®), non-nucleoside reverse transcriptase inhibitors such as delavirdine (Rescriptor®), efavirenz (Sustiva®), nevairapine (Viramune®) and etravirine (Intelence®), nucleotide reverse transcriptase inhibitors such as tenofovir (Viread®), protease inhibitors such as amprenavir (Agenerase®), atazanavir (Reyataz®), darunavir (Prezista®), fosamprenavir (Lexiva®), indinavir (Crixivan®), lopinavir and ritonavir (Kaletra®), nelfinavir (Viracept®), ritonavir (Norvir®), saquinavir (Fortovase® or Invirase®), and tipranavir (Aptivus®), entry inhibitors such as enfuvirtide (Fuzeon®) and maraviroc (Selzentry®), integrase inhibitors such as raltegravir (Isentress®), doxorubicin (Hydrodaunorubicin®), vincristine (Oncovin®), bortezomib (Velcade®), and dexamethasone (Decadron ®) in combination with lenalidomide (Revlimid ®), anti-IL36 agents such as BI655130, Dihydroorotate dehydrogenase inhibitors such as IMU-838, anti-OX40 agents such as KHK-4083, microbiome agents such as RBX2660, SER-287, Narrow spectrum kinase inhibitors such as TOP-1288, anti-CD40 agents such as BI-655064 and FFP-104, guanylate cyclase agonists such as dolcanatide, sphingosine kinase inhibitors such as opaganib, anti-IL-12/IL-23 agents such as AK-101, Ubiquitin protein ligase complex inhibitors such as BBT- 401, sphingosine receptors modulators such as BMS-986166, P38MAPK/PDE4 inhibitors such as CBS-3595, CCR9 antagonists such as CCX-507, FimH antagonists such as EB-8018, HIF-PH inhibitors such as FG-6874, HIF-1α stabilizer such as GB-004, MAP3K8 protein inhibitors such as GS-4875, LAG-3 antibdies such as GSK-2831781, RIP2 kinase inhibitors such as GSK- 2983559, Farnesoid X receptor agonist such as MET-409, CCK2 antagonists such as PNB-001, IL-23 Receptor antagonists such as PTG-200, Purinergic P2X7 receptor antagonists such as SGM-1019, PDE4 inhibiotrs such as Apremilast, ICAM-1 inhibitors such as alicaforsen sodium, Anti- IL23 agents such as guselkumab, brazikumab and mirkizumab, ant-IL-15 agents such as AMG-714, TYK-2 inhibitors such as BMS-986165, NK Cells activators such as CNDO-201, RIP-1 kinase inhibitors such as GSK-2982772, anti-NKGD2 agents such as JNJ-4500, CXCL-10 antibodies such as JT-02, IL-22 receptor agonists such as RG-7880, GATA-3 antagonists such as SB-012 and Colony-stimulating factor-1 receptor inhibitors such as edicotinib or any combination(s) thereof.

### Diseases caused by viruses

A co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, may be useful in the treatment or prevention of various diseases caused by viruses, in particular by retroviruses and more particularly by HIV, and more particularly for use for lowering viral load in a patient infected by a virus, in particular HIV, or a virus-related condition, with a long-lasting effect and absence of resistance.

Examples of viruses which are considered by the invention include enveloped and naked viruses, which includes DNA viruses, RNA viruses and retroviruses, which includes dsDNA viruses, ssDNA viruses, dsRNA viruses, (+)ssRNA viruses, (-)ssRNA viruses, ssRNA-RT viruses and dsDNA-RT viruses.

Viruses which are more particularly considered are RNA viruses and retroviruses, including lentiviruses, and preferably HIV. Accordingly, virus-related conditions which are more particularly considered are associated with a RNA virus or a retrovirus, and preferably HIV. HIV may include HIV-I, HIV-2 and all subtypes thereof, which includes HIV-I strains belonging to the HIV-I B subtype, HIV-I C subtype, and HIV-I recombinants. Examples include HIV-I strains selected from Ad8, AdaM, Isolate B, Isolate C, CRF01, CRF02 and CRF06. According to a preferred embodiment, the virus-related condition is AIDS.

Three subfamilies can be distinguished within the retroviral family: the oncoviruses, the lentiviruses and the spumaviruses. HIV pertains to the lentiviruses.

According to a particular embodiment, the retroviruses include HIV virus (HIV1 and HIV2), visna/maedi virus or MVV/visna, equine infectious anemia virus or EIAV, caprine arthritis encephalitis virus or CAEV, simian immunodeficiency virus or SIV, avian leukemia virus or ALV, murine leukemia virus also called Moloney virus or MULV, Abelson leukemia virus, murine mammary tumor virus, Mason-Pfizer monkey virus or MPMV, feline leukemia virus or FELV, human leukemia viruses HTLV-I, human leukemia viruses HTLV-II, simian leukemia virus or STLV, bovine leukemia virus or BLV, primate type D oncoviruses, type B oncoviruses, Rous sarcoma virus or RSV, simian foamy virus or SFV or chimpanzee simian virus, human foamy virus, and feline immunodeficiency virus, the human foamy virus or HFV, bovine syncytial virus or BSV, feline syncytial virus FSV, the feline immunodeficiency virus, avian leukosis virus, Walleye dermal sarcoma virus, T-cell lymphoma, acute ATL, lymphomatous ATL, chronic ATL, smoldering ATL, neurologic diseases, Tropical spastic paraparesis or HTLV-associated myelopathy, inflammatory and autoimmune diseases such as uveitis, dermatitis, pneumonitis, rheumatoid arthritis, and polymyositis hematologic and dermatologic diseases, lung diseases, brain diseases, and/or immunodeficiency.

As described herein, the term oncovirus can include Alpharetrovirus (for example, avian leukosis virus and Rous sarcoma virus); Betaretrovirus (for example, mouse mammary tumor virus); Gammaretrovirus (for example, murine leukemia virus and feline leukemia virus); Deltaretrovirus (for example bovine leukemia virus and human T-lymphotropic virus); and Epsilonretrovirus (for example, Walleye dermal sarcoma virus).

More generally, the retroviruses described herein may be, for example, visna/maedi virus or MVV/visna, equine infectious anemia virus or EIAV, caprine arthritis encephalitis virus or CAEV, simian immunodeficiency virus or SIV, avian leukemia virus or ALV, murine leukemia virus also called Moloney virus or MULV, Abelson leukemia virus, murine mammary tumor virus, Mason-Pfizer monkey virus or MPMV, feline leukemia virus or FELV, human leukemia viruses HTLV-I, human leukemia viruses HTLV-II, simian leukemia virus or STLV, the bovine leukemia virus or BLV, primate type D oncoviruses, type B oncoviruses, Rous sarcoma virus or RSV, and/or simian foamy virus or SFV or chimpanzee simian virus, human foamy virus, and feline immunodeficiency virus, the human foamy virus (or HFV), bovine syncytial virus (or BSV), feline syncytial virus (FSV) and the feline immunodeficiency virus.

More particularly, HTLV-I causes T-cell lymphoma (ATL for Adult T-cell leukemia/lymphoma, including the different forms of ATL such as acute ATL, lymphomatous ATL, chronic ATL and smoldering ATL), neurologic disease, Tropical spastic paraparesis (TSP) (also known as HTLV-associated myelopathy (HAM) or chronic progressive myelopathy), and diverse inflammatory and autoimmune diseases such as uveitis, dermatitis, pneumonitis, rheumatoid arthritis, and; HTLV-II may play a role in certain neurologic, hematologic and dermatologic diseases; HIV (HIV1 and HIV2) causes AIDS; the visna virus causes lung and brain diseases in sheep; the feline immunodeficiency virus causes immunodeficiency in cat; Rous sarcoma virus and mouse mammary tumor virus causes tumor growth and cancer.

The invention also relates to a method for treating and/or preventing diseases caused by viruses, in particular by retroviruses and more particularly by HIV, and which comprises a step of administering a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention to a patient in need thereof.

Additionally, the present invention has for purpose to lower a viral load in a patient infected by a virus, in particular HIV, or a virus-related condition, with a long-lasting effect and absence of resistance, by using a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention.

In one embodiment, the present invention concerns a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, for use for treating or preventing a retroviral infection or a retrovirus-related condition, in particular a HIV infection or a HIV-related condition in a patient, for which an ineffectiveness or a decline in a prior anti-retroviral treatment effectiveness has been stated.

In another embodiment, the present invention relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, for use for treating or preventing a retroviral infection or a retrovirus-related condition, in particular a HIV infection or a HIV-related condition in a patient, wherein the patient is infected by a drug-resistant viral strain, and more particularly by a drug-resistant HIV strain.

Furthermore, the invention further relates to new doses and regimens of said co-crystal of 8-chloro-N-(4-(trifluoromethoxy)phenyl)quinolin-2-amine as defined in the present invention, or said pharmaceutically acceptable salt of 8-chloro-N-(4-(trifluoromethoxy)phenyl)quinolin-2-amine including a solvate and/or a hydrate thereof as defined in the present invention and use in the treatment or prevention of viral infection, and in particular HIV, or a virus-related condition, more particularly where the use maintains a low viral load after treatment termination. Thus, according to one embodiment, the invention relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use for treating or preventing of a virus infection or virus-related condition in a patient, in particular a HIV infection or a HIV-related condition, wherein: a low or undetectable viral load is maintained; and/or a CD4+ cell count is stable or increased; after treatment termination.

According to another embodiment, the invention relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, for use in the treatment or prevention of a virus infection or virus-related condition in patient, in particular a HIV infection or a HIV-related condition, for which an ineffectiveness in prior anti-retroviral treatment, or a decline in a prior anti-viral, or anti-retroviral, treatment effectiveness has been stated.

According to still another embodiment, the invention relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, for use in the treatment or prevention of a virus infection or virus-related condition in patient, in particular a HIV infection or a HIV-related condition, wherein the patient is infected by a drug-resistant strain.

In the framework of the present invention, the co-crystal of 8-chloro-N-(4-(trifluoromethoxy)phenyl)quinolin-2-amine as defined in the present invention, or the pharmaceutically acceptable salt of 8-chloro-N-(4-(trifluoromethoxy)phenyl)quinolin-2-amine including a solvate and/or a hydrate thereof as defined in the present invention may be administered in combination with another anti-retroviral agent. According to one embodiment, an ART (Antiretroviral Therapy) or HAART (Highly Active Antiretroviral Therapy) may be implemented using one or more of the following antiretroviral compounds:
(i) nucleoside/nucleotide reverse transcriptase inhibitors also called nucleoside analogs, such as abacavir, emtricitabine, and tenofovir;
(ii) non-nucleoside reverse transcriptase inhibitors (NNRTIs), such as efavirenz, etravirine, and nevirapine;
(iii) protease inhibitors (PIs), such as atazanavir, darunavir, and ritonavir;
(iv) entry inhibitors, such as enfuvirtide and maraviroc;
(v) integrase inhibitors, such as dolutegravir and raltegravir.

Other examples of anti-retroviral agents include, in a non-limitative manner: Zidovudine, Lamivudine, Emtricitabine, Didanosine, Stavudine, Abacavir, Zalcitabine, Racivir, Amdoxovir, Apricitabine, Elvucitabine, Efavirenz, Nevirapine, Etravirine, Delavirdine, Rilpvirine, Tenofovir, Fosalvudine, Amprenavir, Tipranavir, Indinavir, Saquinavir, Fosamprenavir, Ritonavir, Darunavir, Atazanavir, Nelfinavir, Lopinavir, Raltegravir, Elvitegravir, Dolutegravir, Enfuvirtide, Maraviroc, Vicriviroc, and combinations thereof.

In some embodiments, a co-crystal of 8-chloro-N-(4-(trifluoromethoxy)phenyl)quinolin-2-amine as defined in the present invention, or a pharmaceutically acceptable salt of 8-chloro-N-(4-(trifluoromethoxy)phenyl)quinolin-2-amine including a solvate and/or a hydrate thereof as defined in the present invention may be administered in combination with one or more additional therapeutic agents selected from nucleoside reverse transcriptase inhibitors such as zidovudine (Retrovir®), abacavir (Ziagen®), abacavir/lamivudine (Epzicom®), abacavir/lamivudine/zidovudine (Trizivir®), didanosine (Videx®), emtricitabine (Emtriva®), lamivudine (Epivir®), lamivudine/zidovudine (Combivir®), stavudine (Zerit®), and zalcitabine (Hivid®), non-nucleoside reverse transcriptase inhibitors such as delavirdine (Rescriptor®), efavirenz (Sustiva®), nevairapine (Viramune®) and etravirine (Intelence®), nucleotide reverse transcriptase inhibitors such as tenofovir (Viread®), protease inhibitors such as amprenavir (Agenerase®), atazanavir (Reyataz®), darunavir (Prezista®), fosamprenavir (Lexiva®), indinavir (Crixivan®), lopinavir and ritonavir (Kaletra®), nelfinavir (Viracept®), ritonavir (Norvir®), saquinavir (Fortovase® or Invirase®), and tipranavir (Aptivus®), entry inhibitors such as enfuvirtide (Fuzeon®) and maraviroc (Selzentry®), integrase inhibitors such as raltegravir (Isentress®), and combinations thereof.

### Cancers

A co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention, may be useful in the treatment or prevention of various cancers.

As used herein the term "cancer", and unless stated otherwise, may relate to any disorder associated with abnormal cell growth, which thus includes malignant tumors and benign tumors, metastatic tumors and non-metastatic tumors, solid tumors and non-solid tumors, such as Blood-Related Cancers which may thus include Leukaemia, Lymphoma and Myeloma; it may also relate to Central Nervous System (CNS) cancers and non-CNS cancers. Unless stated otherwise, the term *"cancer"* also encompasses juvenile and non-juvenile cancers, Recurrent and Non-Recurrent cancers as well as cancer relapses.

Among cancers, the following may be cited: Blood-Related Cancer, pancreatic cancer, urological cancer, bladder cancer, colorectal cancer, colon cancer, breast cancer, prostate cancer, renal cancer, hepatocellular cancer, thyroid cancer, gall bladder cancer, lung cancer (e.g. non-small cell lung cancer, small-cell lung cancer), ovarian cancer, cervical cancer, gastric cancer, endometrial cancer, oesophageal cancer, head and neck cancer, melanoma, neuroendocrine cancer, CNS cancer, brain tumors (e.g., glioma, anaplastic oligodendroglioma, adult glioblastoma multiforme, and adult anaplastic astrocytoma), bone cancer, soft tissue sarcoma, retinoblastomas, neuroblastomas, peritoneal effusions, malignant pleural effusions, mesotheliomas, Wilms tumors, trophoblastic neoplasms, hemangiopericytomas, Kaposi's sarcomas, myxoid carcinoma, round cell carcinoma, squamous cell carcinomas, oesophageal squamous cell carcinomas, oral carcinomas, cancers of the adrenal cortex, or ACTH-producing tumors.

According to one embodiment, the following cancers may be cited: head and neck cancer, stomach cancer, breast cancer, basal and squamous skin cell cancer, liver cancer, kidney cancer, brain cancer, lung cancer, pancreatic cancer, eye cancer, gastrointestinal cancer, colorectal cancer, oesophageal cancer, colorectal cancer, bladder cancer, gall bladder cancer, thyroid cancer, melanoma, uterine/cervical cancer, ovarian, cancer, bone cancer and renal cancer.

According to another embodiment, the cancer may be selected from head and neck cancer, Head and Neck Squamous Cell Carcinoma, Neck Squamous Cell Carcinoma, Acute Lymphocytic Leukemia (ALL) in Adults or children, Acute Myeloid Leukemia (AML) in adults or children, Acute Lymphoblastic Leukemia, Adrenal Cancer, Anal Cancer, Astrocytic Glioma, Astrocytoma (grade I, II, III, or IV), B- or NK/T-cell lymphomas, Basal and Squamous Skin Cell Cancer, Bile Duct Cancer, Bladder Cancer, Bone Cancer, brain cancer, Brain and Spinal Cord Tumors in Adults, Brain and Spinal Cord Tumors in Children, Anaplastic astrocytomas, Breast cancer, Gastrointestitnal cancer, Breast Cancer in Women, Breast Cancer in Young Women, Breast Cancer in Men, Recurrent Breast Cancer, Hereditary Breast Cancer, HER2 positive Breast Cancer, Breast Cancer associated with lymph node metastatis, ER-alpha positive Breast Cancer, Cancer in Adolescents, Cancer in Children, Cancer in Young Adults, Cancer of Unknown Primary, Castleman Disease, Cervical Cancer, Cervical Intraepithelial Neoplasia, Cholangiocarcinoma, Chronic Lymphocytic Leukemia (CLL), Chronic Myeloid Leukemia (CML), Chronic Myelomonocytic Leukemia (CMML), Colorectal Cancer, colorectal adenoma, Cutaneous Squamous Cell Carcinoma, Endometrial Cancer, Epithelial Ovarian Cancer, Epithelial Ovarian Cancer associated with metastasis, esophageal cancer, esophagus Squamous Cell Carcinoma, Ewing sarcoma, Ewing Family of Tumors, Lymphoblastic leukaemia (ALL), Eye Cancer, such as Ocular Melanoma and Lymphoma, Gallbladder Cancer, Gastric Cancer, Gastrointestinal Carcinoid Tumors, Gastrointestinal Stromal Tumor (GIST), Gestational Trophoblastic Disease, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, Hodgkin Lymphoma, Kaposi Sarcoma, Kidney Cancer, Laryngeal and Hypopharyngeal Cancer, Leiomyosarcoma, Leukemia, Leukemia in Children, Liver Cancer, Lung Cancer, Lung Carcinoid Tumor, Lymphoma, Lymphoma of the Skin, Malignant Mesothelioma, Mantle cell lymphoma, Medulloblastoma, Melanoma Skin Cancer, malignant melanoma, Meningioma, Merkel Cell Skin Cancer, Multiple Myeloma, Multiple Myeloma with Osteonecrosis of the Jaw, Myelodysplastic Syndrome, Nasal Cavity and Paranasal Sinuses Cancer, Nasopharyngeal Cancer, recurrent or metastatic Nasopharyngeal carcinoma, Neuroblastoma, Neuroglioma, Non-Hodgkin Lymphoma, Non-Hodgkin Lymphoma in Children, Non-Small Cell Lung Cancer, Gefitinib-resistant non-small cell lung cancer, Oral cancer, Oral Cavity and Oropharyngeal Cancer, Osteosarcoma, Pulmonary Metastatic Osteosarcoma, Ovarian Cancer, Pancreatic Cancer, thyroid carcinoma, Papillary Thyroid Carcinoma, Pediatric Spinal Ependymoma, Penile Cancer, Pituitary Tumors, Pituitary Adenoma, Proneural tumors, Prostate Cancer, Retinoblastoma, Rhabdomyosarcoma, Salivary Gland Cancer, Skin Cancer, Small Cell Lung Cancer, Small Intestine Cancer, Soft Tissue Sarcoma, Squamous Cell Carcinoma of the Tongue, Stomach Cancer, Testicular Cancer, Thymus Cancer, Thyroid Cancer, Uterine Sarcoma, Vaginal Cancer, Vulvar Cancer, Renal cancer, Retinoblastoma, Waldenstrom Macroglobulinemia and Wilms Tumor.

According to another embodiment, the cancer may be selected from a Head and Neck Squamous Cell Carcinoma, Neck Squamous Cell Carcinoma, Acute Lymphocytic Leukemia (ALL) in Adults or children, Acute Myeloid Leukemia (AML) in adults or children, Acute Lymphoblastic Leukemia, Adrenal Cancer, Anal Cancer, Astrocytic Glioma, Astrocytoma (grade I, II, III, or IV), B- or NK/T-cell lymphomas, Basal and Squamous Skin Cell Cancer, Bile Duct Cancer, Bone Cancer, brain cancer, Brain and Spinal Cord Tumors in Adults, Brain and Spinal Cord Tumors in Children, Anaplastic astrocytomas, Gastrointestitnal cancer, Breast Cancer in Women, Breast Cancer in Young Women, Breast Cancer in Men, Recurrent Breast Cancer, Hereditary Breast Cancer, HER2 positive Breast Cancer, Breast Cancer associated with lymph node metastatis, ER-alpha positive Breast Cancer, Cancer in Adolescents, Cancer in Children, Cancer in Young Adults, Cancer of Unknown Primary, Castleman Disease, Cervical Intraepithelial Neoplasia, Cholangiocarcinoma, Chronic Lymphocytic Leukemia (CLL), Chronic Myeloid Leukemia (CML), Chronic Myelomonocytic Leukemia (CMML), colorectal adenoma, Cutaneous Squamous Cell Carcinoma, Endometrial Cancer, Epithelial Ovarian Cancer, Epithelial Ovarian Cancer associated with metastasis, esophagus Squamous Cell Carcinoma, Ewing sarcoma, Ewing Family of Tumors, Lymphoblastic leukaemia (ALL), Eye Cancer, such as Ocular Melanoma and Lymphoma, Gastric Cancer, Gastrointestinal Carcinoid Tumors, Gastrointestinal Stromal Tumor (GIST), Gestational Trophoblastic Disease, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, Hodgkin Lymphoma, Kaposi Sarcoma, Laryngeal and Hypopharyngeal Cancer, Leiomyosarcoma, Leukemia, Leukemia in Children, Lung Carcinoid Tumor, Lymphoma, Lymphoma of the Skin, Malignant Mesothelioma, Mantle cell lymphoma, Medulloblastoma, malignant melanoma, Meningioma, Merkel Cell Skin Cancer, Multiple Myeloma, Multiple Myeloma with Osteonecrosis of the Jaw, Myelodysplastic Syndrome, Nasal Cavity and Paranasal Sinuses Cancer, Nasopharyngeal Cancer, recurrent or metastatic Nasopharyngeal carcinoma, Neuroblastoma, Neuroglioma, Non-Hodgkin Lymphoma, Non-Hodgkin Lymphoma in Children, Gefitinib-resistant non-small cell lung cancer, Oral cancer, Oral Cavity and Oropharyngeal Cancer, Osteosarcoma, Pulmonary Metastatic Osteosarcoma, thyroid carcinoma, Papillary Thyroid Carcinoma, Pediatric Spinal Ependymoma, Penile Cancer, Pituitary Tumors, Pituitary Adenoma, Proneural tumors, Retinoblastoma, Rhabdomyosarcoma, Salivary Gland Cancer, Skin Cancer, Small Cell Lung Cancer, Small Intestine Cancer, Soft Tissue Sarcoma, Squamous Cell Carcinoma of the Tongue, Testicular Cancer, Thymus Cancer, Uterine Sarcoma, Vaginal Cancer, Vulvar Cancer, Renal cancer, Retinoblastoma, Waldenstrom Macroglobulinemia and Wilms Tumor.

According to a further embodiment, the cancer may be selected from head and neck cancer, Head and Neck Squamous Cell Carcinoma, Neck Squamous Cell Carcinoma, Malignant melanoma, stomach cancer, Breast cancer, Breast cancer in Women, Breast Cancer in Young Women, basal and squamous skin cell cancer, liver cancer, brain cancer, Anaplastic astrocytomas, lung cancer, Non-Small Cell Lung Cancer, Gefitinib- resistant non-small cell lung cancer, Oral cancer, eye cancer, Gastric Cancer, gastrointestinal cancer, Astrocytic Glioma, Astrocytoma (grade I, II, III, or IV), colorectal cancer, colorectal adenoma, Cutaneous Squamous Cell Carcinoma, bladder cancer, bone cancer, Recurrent Breast Cancer, Hereditary Breast Cancer, HER2 positive Breast Cancer, Breast Cancer associated with lymph node metastatis, ER-alpha positive Breast Cancer, renal cancer, Cervical Intraepithelial Neoplasia, Cholangiocarcinoma, Leiomyosarcoma, Chronic Lymphocytic Leukemia (CLL), Chronic Myeloid Leukemia (CML), Chronic Myelomonocytic Leukemia (CMML), Acute Myeloid Leukemia (AML) in adults or children, Acute Lymphoblastic Leukemia,, B- or NK/T-cell lymphomas, cervical cancer, Glioblastoma, Glioblastoma multiforme (GBM), Hairy cell leukemia, Glioma, High-grade glioma, Hepatocellular carcinoma, Intrahepatic cholangiocarcinoma, Invasive Breast Ductal Carcinoma, kidney cancer, Endometrial cancer, ovarian cancer, Epithelial Ovarian Cancer, Epithelial Ovarian Cancer associated with metastasis, esophageal cancer, esophageal Squamous Cell Carcinoma, Ewing sarcoma, Lymphoblastic leukaemia (ALL), Mantle cell lymphoma, Medulloblastoma, Lymphoma, Myelodysplastic syndrome, Meningioma, Multiple Myeloma (MM), Multiple Myeloma with Osteonecrosis of the Jaw, Nasopharyngeal Cancer, recurrent or metastatic Nasopharyngeal carcinoma, Neuroblastoma, Neuroglioma, Papillary Thyroid Carcinoma, Pediatric Spinal Ependymoma, Osteosarcoma, Pulmonary Metastatic Osteosarcoma, pancreatic cancer, thyroid carcinoma, sarcoma, pituitary tumors, Pituitary Adenoma, Proneural tumors, Squamous Cell Carcinoma of the Tongue, Mesothelioma, Retinoblastoma and prostate cancer.

According to a further embodiment, the cancer may be selected from Head and Neck cancer, Head and Neck Squamous Cell Carcinoma, Neck Squamous Cell Carcinoma, malignant melanoma, Astrocytic Glioma, Glioma, stomach cancer, Breast cancer, Cholangiocarcinoma, recurrent or metastatic Nasopharyngeal carcinoma, basal and squamous skin cell cancer, liver cancer, brain cancer, Anaplastic astrocytomas, lung cancer, Non-Small Cell Lung Cancer, Gefitinib-resistant non-small cell lung cancer, Oral cancer, Glioblastoma, osteosarcoma, Pulmonary Metastatic Osteosarcoma, pancreatic cancer, eye cancer, gastrointestinal cancer, colorectal cancer, colorectal adenoma, Cutaneous Squamous Cell Carcinoma, Endometrial cancer, Epithelial Ovarian Cancer, esophageal cancer, Ewing sarcoma, gastric cancer, Hepatocellular carcinoma, HER2 positive Breast Cancer, bladder cancer, bone cancer, prostate cancer, Retinoblastoma and renal cancer.

According to a further embodiment, the cancer may be selected from Anaplastic astrocytomas, Astrocytic gliomas, Bladder cancer, Breast cancer, Cholangiocarcinoma, Colorectal cancer, Colorectal adenoma, Cutaneous squamous cell carcinoma, Endometrial cancer, Epithelial ovarian cancer, Esophageal cancer, Ewing sarcoma, Gastric cancer, Gefitinib-resistant non-small cell lung cancer, Glioblastoma, Glioma, Hepatocellular carcinoma, HER2 positive breast cancer, Head and Neck Squamous Cell Carcinoma, Malignant melanoma, Nasopharyngeal carcinoma (recurrence or metastasis), Neck squamous cell carcinoma, Non-small cell lung cancer, Oral cancer, Osteosarcoma, Osteosarcoma (pulmonary metastasis), Prostate cancer and retinoblastoma

According to a further embodiment, the cancer may be selected from anal cancer, bile duct cancer, gastrointestinal cancer, Cholangiocarcinoma, colorectal cancer, colorectal adenoma, esophageal cancer, Esophagus Squamous Cell Carcinoma ,gastric cancer, Gastrointestinal Carcinoid Tumors, Gastrointestinal Stromal Tumor (GIST), Hepatocellular carcinoma, Intrahepatic cholangiocarcinoma, liver cancer, lung cancer, Lung Carcinoid Tumor, non-Small Cell Lung Cancer, Gefitinib-resistant non-small cell lung cancer, Pulmonary Metastatic Osteosarcoma, stomach cancer, pancreatic cancer, Small Cell Lung Cancer, and Small Intestine Cancer

According to one embodiment, the patient does not present clinically detectable metastases, in particular said patient has a pre-cancerous condition, an early stage cancer or a non-metastatic cancer, or said patient presents clinically detectable metastases and said co-crystal of 8-chloro-N-(4-(trifluoromethoxy)phenyl)quinolin-2-amine as defined in the present invention, or said pharmaceutically acceptable salt of 8-chloro-N-(4-(trifluoromethoxy)phenyl)quinolin-2-amine including a solvate and/or a hydrate thereof as defined in the present invention does not target directly the invasion of metastases.

In view of the above, the invention relates to a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use in the treatment and/or prevention of cancer, such as the here above listed cancers, and dysplasia.

Thus, the invention also relates to the use of a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for treating and/or preventing cancer, such as the here above listed cancers, and dysplasia.

The invention also relates to the use of a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention for the preparation of a composition, such as a medicament, for treating and/or preventing of cancer, such as the here above listed cancers, and dysplasia.

The invention also relates to a method of preventing, inhibiting or treating cancer or dysplasia, which comprises at least one step consisting in administering to a patient suffering therefrom an effective amount of a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention.

In some embodiments, the invention relates to a method of the present invention or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, for treating and/or preventing cancer or dysplasia, wherein a presence and/or expression level of miR-124 in a blood and/or tissue sample of the patient, is measured prior to and/or during the use.

In some embodiments, the invention relates to a method of the present invention or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, for treating and/or preventing cancer or dysplasia, wherein a presence and/or expression level of miR-124 in a blood and/or tissue sample is measured to guide dose or monitor response to the treatment.

In some embodiments, the invention relates to a method of the present invention or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, for treating and/or preventing cancer or dysplasia, wherein the level of a co-crystal of 8-chloro-N-(4-(trifluoromethoxy)phenyl)quinolin-2-amine as defined in the present invention, or of a pharmaceutically acceptable salt of 8-chloro-N-(4-(trifluoromethoxy)phenyl)quinolin-2-amine including a solvate and/or a hydrate thereof as defined in the present invention, in a blood, plasma, tissue, saliva, and/or serum sample of the patient is measured during the use.

In some embodiments, the invention relates to a method of the present invention or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, for treating and/or preventing cancer or dysplasia, which is used in combination with another anti-tumoral agent.

In some embodiments, the invention relates to a method of the present invention or a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as defined above, for treating and/or preventing cancer or dysplasia, which is used in combination with another therapy selected from chemotherapy, immunotherapy, radiotherapy, surgery, ultrasounds, monoclonal antibodies, and cancer vaccines.

Among other anticancer drug, the following may be cited:
- Androgen receptor inhibitors, such as enzalutamide (Xtandi®, Astellas/Medivation), abiraterone (Zytiga®, Centocor/Ortho), antagonist of gonadotropin-releasing hormone (GnRH) receptor such as degaralix, Firmagon®, Ferring Pharmaceuticals)
- **Antiapoptotics,** such as venetoclax (Venclexta®, AbbVie/Genentech), blinatumomab (Blincyto®, Amgen), navitoclax (ABT-263, Abbott);
- **Antiproliferative and Antimitotic agents,** such as vinca alkaloids (which include vinblastine, vincristine);
- **Antibiotics** such as dactinomycin, daunorubicin, doxorubicin, idarubicin, anthracyclines, mitoxantrone, bleomycins, plicamycin (mithramycin), and mitomycin;
- **L-asparaginase;**
- **Antiplatelet agents;**
- **Antiproliferative/antimitotic alkylating agents** such as nitrogen mustards cyclophosphamide and analogs (which include melphalan, chlorambucil, hexamethylmelamine, and thiotepa), alkyl nitrosoureas (which include carmustine) and analogs, streptozocin, and triazenes (which include dacarbazine);
- **Antiproliferative/antimitotic antimetabolites** such as folic acid analogs (which include methotrexate), aromatase inhibitors; antiestrogens; topoisomerase I inhibitors; topoisomerase II inhibitors; microtubule active compounds; alkylating compounds; histone deacetylase inhibitors; compounds which induce cell differentiation processes; cyclooxygenase inhibitors; MMP inhibitors; mTOR inhibitors; antineoplastic

antimetabolites; platin compounds; compounds targeting/decreasing a protein or lipid kinase activity and further anti-angiogenic compounds; compounds which target, decrease or inhibit the activity of a protein or lipid phosphatase; gonadorelin agonists; anti-androgens; methionine aminopeptidase inhibitors; matrix metalloproteinase inhibitors; bisphosphonates; biological response modifiers; antiproliferative antibodies; heparanase inhibitors; inhibitors of Ras oncogenic isoforms; telomerase inhibitors; proteasome inhibitors; compounds used in the treatment of hematologic malignancies; compounds which target, decrease or inhibit the activity of Flt-3; Hsp90 inhibitors such as 17-AAG (17-allylaminogeldanamycin, NSC330507), 17-DMAG (17-dimethylaminoethylamino-17-demethoxy-geldanamycin, NSC707545), IPI-504, CNF1010, CNF2024, CNF1010 from Conforma Therapeutics; temozolomide (Temodal®); kinesin spindle protein inhibitors, such as SB715992 or SB743921 from GlaxoSmithKline, or pentamidine/chlorpromazine from CombinatoRx; MEK inhibitors such as ARRY142886 from Array BioPharma, AZd6244 from AstraZeneca, PD181461 from Pfizer and leucovorin;
- **Antimigratory agents;**
- **Angiogenesis inhibitors,** such as TNP-470;
- **Aromatase inhibitors,** such as letrozole and anastrozole, exemestane;
- **Angiotensin**
- **Anti-sense oligonucleotides,** such as antisense nucleic acids directed toward miR-124;
- **Anticoagulants,** such as heparin, synthetic heparin salts, and other inhibitors of thrombin;
- **Arginine inhibitors,** such as AEB1102 (pegylated recombinant arginase, Aeglea Biotherapeutics) and CB-1158 (Calithera Biosciences);
- **Bone resorption inhibitors,** such as Denosumab (Xgeva®, Amgen), bisphosphonates such as zoledronic acid (Zometa®, Novartis);
- **CC chemokine receptor 4 (CCR4) inhibitors,** such as mogamulizumab (Poteligeo®, Kyowa Hakko Kirin, Japan);
- **CDK inhibitors,** such as CDK4/CDK6 inhibitors, such as palbociclib (Ibrance®, Pfizer); ribociclib (Kisqali®, Novartis); abemaciclib (Ly2835219, Eli Lilly); and trilaciclib (G1T28, G1 Therapeutics) ;
- **Cell cycle inhibitors and differentiation inducers,** such as as tretinoin;
- **Corticosteroids,** such as cortisone, dexamethasone, hydrocortisone, methylprednisolone, prednisone, and prednisolone;
- **DNA damaging agents** such as actinomycin, amsacrine, busulfan, carboplatin, chlorambucil, cisplatin, cyclophosphamide (CYTOXAN®), dactinomycin, daunorubicin, doxorubicin, epirubicin, iphosphamide, melphalan, merchlorethamine, mitomycin, mitoxantrone, nitrosourea, procarbazine, taxol, taxotere, teniposide, etoposide, and triethylenethiophosphoramide;
- **Fibrinolytic agents,** such as tissue plasminogen activator, streptokinase, urokinase, aspirin, dipyridamole, ticlopidine, and clopidogrel;
- **Folate antagonists;**
- **FLT3 receptor inhibitors,** such as enzalutamide, abiraterone, apalutamide, erlotinib, crizotinib, niraparib, olaparib, osimertinib, regorafenib, sunitinib, lestaurtinib, midostaurin, gilteritinib, semaxinib, linifanib, fostamatinib, pexidartinib, sorafenib, cabozantinib, ponatinib, ilorasertib, pacritinib, famitinib, pexidartinib, quizartinib;
- **Glutaminase inhibitors,** such as CD-839 (Calithera Biosciences);
- **Growth Factor Signal transduction kinase inhibitors;**
- **Growth factor Inhibitors,** such as vascular endothelial growth factor inhibitors and fibroblast growth factor inhibitors, such as olaratumab (Lartruvo®; Eli Lilly), cetuximab (Erbitux®, Eli Lilly); necitumumab (Portrazza®, Eli Lilly), panitumumab (Vectibix®, Amgen); and osimertinib (targeting activated EGFR, Tagrisso®, AstraZeneca);
- **Hedgehog pathway inhibitors,** such as sonidegib (Odomzo®, Sun Pharmaceuticals); and vismodegib (Erivedge®, Genentech);
- **Histone deacetylase (HDAC) inhibitors,** such as vorinostat (Zolinza®, Merck); romidepsin (Istodax®, Celgene); panobinostat (Farydak®, Novartis); belinostat (Beleodaq®, Spectrum Pharmaceuticals); entinostat (SNDX-275, Syndax Pharmaceuticals) (NCT00866333); and chidamide (Epidaza®, HBI-8000, Chipscreen Biosciences, China);
- **Hormones and analogs thereof,** such as estrogen, tamoxifen, goserelin, bicalutamide, and nilutamide);
- **Isocitrate dehydrogenase (IDH) inhibitors,** such as AG120 (Celgene; NCT02677922); AG221 (Celgene, NCT02677922; NCT02577406); BAY1436032 (Bayer, NCT02746081); IDH305 (Novartis, NCT02987010)
- **Isoflavones** such as genistein;
- **Immunosuppressives,** such as tacrolimus, sirolimus, azathioprine, and mycophenolate;
- **Inhibitors of p53 suppressor proteins,** such as ALRN-6924 (Aileron);
- **Inhibitors of transforming growth factor-beta (TGF-beta or TGFβ),** such as NIS793 (Novartis), fresolimumab (GC1008; Sanofi-Genzyme), M7824 (Merck KgaA - formerly MSB0011459X);
- iNKT cell agonists a such as ABX196 5Abivax)
- **mTOR inhibitors,** such as everolimus (Afinitor®, Novartis); temsirolimus (Torisel®, Pfizer); and sirolimus (Rapamune®, Pfizer) ;
- **Microtubule-inhibiting drugs,** such as taxanes (which include paclitaxel, docetaxel), vinblastin, nocodazole, epothilones, vinorelbine) (NAVELBINE®), and epipodophyllotoxins (etoposide, teniposide);
- **Nitric oxide donors;**
- **Nucleoside inhibitors,** such as trabectedin (guanidine alkylating agent, Yondelis®, Janssen Oncology), mechlorethamine (alkylating agent, Valchlor®, Aktelion Pharmaceuticals); vincristine (Oncovin®, Eli Lilly; Vincasar®, Teva Pharmaceuticals; Marqibo®, Talon Therapeutics); temozolomide (prodrug to alkylating agent 5-(3-methyltriazen-1-yl)-imidazole-4-carboxamide (MTIC) Temodar®, Merck); cytarabine injection (ara-C, antimetabolic cytidine analog, Pfizer); lomustine (alkylating agent, CeeNU®, Bristol-Myers Squibb; Gleostine®, NextSource Biotechnology); azacitidine (pyrimidine nucleoside analog of cytidine, Vidaza®, Celgene); omacetaxine mepesuccinate (cephalotaxine ester) (protein synthesis inhibitor, Synribo®; Teva Pharmaceuticals); asparaginase *Erwinia chrysanthemi* (enzyme for depletion of asparagine, Elspar®, Lundbeck; Erwinaze®, EUSA Pharma); eribulin mesylate (microtubule inhibitor, tubulin-based antimitotic, Halaven®, Eisai); cabazitaxel (microtubule inhibitor, tubulin-based antimitotic, Jevtana®, Sanofi-Aventis); capacetrine (thymidylate synthase inhibitor, Xeloda®, Genentech); bendamustine (bifunctional mechlorethamine derivative, believed to form interstrand DNA cross-links, Treanda®, Cephalon/Teva); ixabepilone (semi-synthetic analog of epothilone B, microtubule inhibitor, tubulin-based antimitotic, Ixempra®, Bristol-Myers Squibb); nelarabine (prodrug of deoxyguanosine analog, nucleoside metabolic inhibitor, Arranon®, Novartis); clorafabine (prodrug of ribonucleotide reductase inhibitor, competitive inhibitor of deoxycytidine, Clolar®, Sanofi-Aventis); and trifluridine and tipiracil (thymidine-based nucleoside analog and thymidine phosphorylase inhibitor, Lonsurf®, Taiho Oncology);
- **PI3K inhibitors,** such as idelalisib (Zydelig®, Gilead), alpelisib (BYL719, Novartis), taselisib (GDC-0032, Genentech/Roche); pictilisib (GDC-0941, Genentech/Roche); copanlisib (BAY806946, Bayer); duvelisib (formerly IPI-145, Infinity Pharmaceuticals); PQR309 (Piqur Therapeutics, Switzerland); and TGR1202 (formerly RP5230, TG Therapeutics);
- **Platinum coordination complexes** (such as cisplatin, oxiloplatin, carboplatin, nedaplatin, picoplatin, procarbazine, mitotane, satraplatin and aminoglutethimide;
- **Poly ADB ribose polymerase (PARP) inhibitor,** such as those selected from: olaparib (Lynparza®, AstraZeneca); rucaparib (Rubraca®, Clovis Oncology); niraparib (Zejula®, Tesaro); talazoparib (MDV3800/BMN 673/LT00673, Medivation/Pfizer/Biomarin); veliparib (ABT-888, AbbVie); and BGB-290 (BeiGene, Inc.) ;
- **Proteasome inhibitors,** such as everolimus (Afinitor®, Novartis); temsirolimus (Torisel®, Pfizer); and sirolimus (Rapamune®, Pfizer), bortezomib (Velcade®, Takeda); carfilzomib (Kyprolis®, Amgen); and ixazomib (Ninlaro®, Takeda);
- **Pyrimidine & Purine analogs,** such as floxuridine, capecitabine, and cytarabine;
- **Receptor blockers, Antisecretory agents,** such as breveldin;
- **Selective estrogen receptor modulator (SERM),** such as raloxifene (Evista®, Eli Lilly);
- **Therapeutic antibodies,** such as those selected from: anti-TNF antibodies, anti-VEGF antibodies, anti-EGFR antibodies, anti-PD-1 antibodies, anti-HER2 antibodies, anti-CD20 antibodies, anti-IL17 antibodies, and anti-CTLA4 antibodies, anti-PDL1, anti-CD25, anti-α4integrin, anti-IL6R, anti-C5, anti-IL1, anti-TPO, anti-IL12/23, anti-EPCAM/CD3, anti-CD30, anti-CD80/86, anti-anthrax, anti-CCR4, anti-CD6, anti-CD19, anti-a4β7, anti-IL6, anti-VEGFR-2, anti-SLAMF7, anti-GD2, anti-IL17A, anti-PCSK9, anti-IL5, anti-CD22, anti-IL4, anti-PDGFRa, anti-IL17RA and anti-TcdB, and such as those selected from: Abagovomab, Abatacept, Abciximab, Abituzumab, Abrilumab, Actoxumab, Adalimumab, Adecatumab, Aducanumab, Aflibercept, Afutuzymab, Alacizumab, Alefacept, Alemtuzumab, Alirocumab, Altumomab, Amatixumab, Anatumomab, Anetumab, Anifromumab, Anrukinzumab, Apolizumab, Arcitumomab, Ascrinvacumab, Aselizumab, Atezolizumab, Atinumab, Altizumab, Atorolimumab, Bapineuzumab, Basiliximab, Bavituximab, Bectumomab, Begelomab, Belatacept, Belimumab, Benralizumab, Bertilimumab, Besilesomab, Bevacizumab, Bezlotoxumab, Biciromab, Bimagrumab, Bimekizumab, Bivatuzumab, Blinatumomab, Blosozumab, Bococizumab, Brentuximab, Briakimumab, Brodalumab, Brolucizumab, Bronticizumab, Canakinumab, Cantuzumab, Caplacizumab, Capromab, Carlumab, Catumaxomab, Cedelizumab, Certolizumab, Cetixumab, Citatuzumab, Cixutumumab, Clazakizumab, Clenoliximab, Clivatuzumab, Codrituzumab, Coltuximab, Conatumumab, Concizumab, Crenezumab, Dacetuzumab, Daclizumab, Dalotuzumab, Dapirolizumab, Daratumumab, Dectrekumab, Demcizumab, Denintuzumab, Denosumab, Derlotixumab, Detumomab, Dinutuximab, Diridavumab, Dorlinomab, Drozitumab, Dupilumab, Durvalumab, Dusigitumab, Ecromeximab, Eculizumab, Edobacomab, Edrecolomab, Efalizumab, Efungumab, Eldelumab, Elgemtumab, Elotuzumab, Elsilimomab, Emactuzumab, Emibetuzumab, Enavatuzumab, Enfortumab, Enlimomab, Enoblituzumab, Enokizumab, Enoticumab, Ensituximab, Epitumomab, Epratuzomab, Erlizumab, Ertumaxomab, Etanercept, Etaracizumab, Etrolizumab, Evinacumab, Evolocumab, Exbivirumab, Fanolesomab, Faralimomab, Farletuzomab, Fasimumab, Felvizumab, Fezkimumab, Ficlatuzumab, Figitumumab, Firivumab, Flanvotumab, Fletikumab,Fontolizumab, Foralumab, Foravirumab, Fresolimumab, Fulramumab, Futuximab, Galiximab, Ganitumab, Gantenerumab, Gavilimomab, Gemtuzumab, Gevokizumab, Girentuximab, Glembatumumab, Golimumab, Gomiliximab, Guselkumab, Ibalizumab, Ibritumomab, Icrucumab, Idarucizumab, Igovomab, Imalumab, Imciromab, Imgatuzumab, Inclacumab, Indatuximab, Indusatumab, Infliximab, Intetumumab, Inolimomab, Inotuzumab, Ipilimumab, Iratumumab, Isatuximab, Itolizumab, Ixekizumab, Keliximab, Labetuzumab, Lambrolizumab, Lampalizumab, Lebrikizumab, Lemalesomab, Lenzilumab, Lerdelimumab, Lexatumumab, Libivirumab, Lifastuzumab, Ligelizumab, Lilotomab, Lintuzumab, Lirilumab, Lodelcizumab, Lokivetmab, Lorvotuzumab, Lucatumumab, Lulizumab, Lumiliximab, Lumretuzumab, Mapatumumab, Margetuximab, Maslimomab, Mavrilimumab, Matuzumab, Mepolizumab, Metelimumab, Milatuzumab, Minetumomab, Mirvetuximab, Mitumomab, Mogamulizumab, Morolimumab, Motavizumab, Moxetumomab, Muromonab-CD3, Nacolomab, Namilumab, Naptumomab, Narnatumab, Natalizumab, Nebacumab, Necitumumab, Nemolizumab, Nerelimomab, Nesvacumab, Nimotuzumab, Nivolumab, Nofetumomab, Obiltoxaximab, Obinutuzumab, Ocaratuzumab, Ocrelizumab, Odulimomab, Ofatumumab, Olaratumab, Olokizumab, Omalizumab, Onartuzumab, Ontuxizumab, Opicinumab, Oportuzumab, Oregovomab, Orticumab, Otelixizumab, Oltertuzumab, Oxelumab, Ozanezumab, Ozoralizumab, Pagibaximab, Palivizumab, Panitumumab, Pankomab, Panobacumab, Parsatuzumab, Pascolizumab, Pasotuxizumab, Pateclizumab, Patritumab, Pembrolizumab, Pemtumomab, Perakizumab, Pertuzumab, Pexelizumab, Pidilizumab, Pinatuzumab, Pintumomab, Polatuzumab, Ponezumab, Priliximab, Pritumumab, Quilizumab, Racotumomab, Radretumab, Rafivirumab, Ralpancizumab, Ramucirumab, Ranibizumab, Raxibacumab, Refanezumab, Regavirumab, Reslizumab, Rilonacept, Rilotumumab, Rinucumab, Rituximab, Robatumumab, Roledumab, Romosozumab, Rontalizumab, Rovelizumab, Ruplizumab, Sacituzumab, Samalizumab, Sarilumab, Satumomab, Secukimumab, Seribantumab, Setoxaximab, Sevirumab, Sibrotuzumab, Sifalimumab, Siltuximab, Siplizumab, Sirukumab, Sofituzumab, Solanezumab, Solitomab, Sonepcizumab, Sontuzumab, Stamulumab, Sulesomab, Suvizumab, Tabalumab, Tacatuzumab, Tadocizumab, Talizumab, Tanezumab, Taplitumomab, Tarextumab, Tefibazumab, Telimomab aritox, Tenatumomab, Teneliximab, Teplizumab, Tesidolumab, TGN 1412, Ticlimumab, Tildrakizumab, Tigatuzumab, TNX-650, Tocilizumab, Toralizumab, Tosatoxumab, Tositumomab, Tovetumab, Tralokimumab, Trastuzumab, TRBS07, Tregalizumab, Tremelimumab, Trevogrumab, Tucotuzumab, Tuvirumab, Ublituximab, Ulocuplumab, Urelumab, Urtoxazumab, Ustekimumab, Vandortuzumab, Vantictumab, Vanucizumab, Vapaliximab, Varlimumab, Vatelizumab, Vedolizumab, Veltuzumab, Vepalimomab, Vesencumab, Visilizumab, Volocixumab, Vorsetuzumab, Votumumab, Zalutumimab, Zanolimumab, Zatuximab, Ziralimumab, Ziv-Aflibercept, and Zolimomab;
- **Topoisomerase inhibitors,** such as doxorubicin, daunorubicin, dactinomycin, eniposide, epirubicin, etoposide, idarubicin, irinotecan, mitoxantrone, topotecan, and irinotecan;
- **Toxins,** such as Cholera toxin, ricin, *Pseudomonas* exotoxin, *Bordetella pertussis* adenylate cyclase toxin, diphtheria toxin, and caspase activators;
**Kinase or VEGF inhibitors,** such as regorafenib (Stivarga®, Bayer); vandetanib (Caprelsa®, AstraZeneca); axitinib (Inlyta®, Pfizer); and lenvatinib (Lenvima®, Eisai); Raf inhibitors, such as sorafenib (Nexavar®, Bayer AG and Onyx); dabrafenib (Tafinlar®, Novartis); and vemurafenib (Zelboraf®, Genentech/Roche); MEK inhibitors, such as cobimetanib (Cotellic®, Exelexis/Genentech/Roche); trametinib (Mekinist®, Novartis); Bcr-Abl tyrosine kinase inhibitors, such as imatinib (Gleevec®, Novartis); nilotinib (Tasigna®, Novartis); dasatinib (Sprycel®, BristolMyersSquibb); bosutinib (Bosulif®, Pfizer); and ponatinib (Inclusig®, Ariad Pharmaceuticals); Her2 and EGFR inhibitors, such as gefitinib (Iressa®, AstraZeneca); erlotinib (Tarceeva®, Genentech/Roche/Astellas); lapatinib (Tykerb®, Novartis); afatinib (Gilotrif®, Boehringer Ingelheim); osimertinib (targeting activated EGFR, Tagrisso®, AstraZeneca); and brigatinib (Alunbrig®, Ariad Pharmaceuticals); c-Met and VEGFR2 inhibitors, such as cabozanitib (Cometriq®, Exelexis); and multikinase inhibitors, such as sunitinib (Sutent®, Pfizer); pazopanib (Votrient®, Novartis); ALK inhibitors, such as crizotinib (Xalkori®, Pfizer); ceritinib (Zykadia®, Novartis); and alectinib (Alecenza®, Genentech/Roche); Bruton's tyrosine kinase inhibitors, such as ibrutinib (Imbruvica®, Pharmacyclics/Janssen); and Flt3 receptor inhibitors, such as midostaurin (Rydapt®, Novartis), tivozanib (Aveo Pharmaecuticals); vatalanib (Bayer/Novartis); lucitanib (Clovis Oncology); dovitinib (TKI258, Novartis); Chiauanib (Chipscreen Biosciences); CEP-11981 (Cephalon); linifanib (Abbott Laboratories); neratinib (HKI-272, Puma Biotechnology); radotinib (Supect®, IY5511, Il-Yang Pharmaceuticals, S. Korea); ruxolitinib (Jakafi®, Incyte Corporation); PTC299 (PTC Therapeutics); CP-547,632 (Pfizer); foretinib (Exelexis, GlaxoSmithKline); quizartinib (Daiichi Sankyo) and motesanib (Amgen/Takeda);

In a non-limitative manner, co-crystals or salts of the invention may be combined, alone or in the form of a kit-of-parts, to one or more of the following anti-cancer drugs or compounds: ABVD, AC, ACE, Abiraterone (Zytiga®), Abraxane, Abstral, Actinomycin D, Actiq, Adriamycin, Afatinib (Giotrif®), Afinitor, Aflibercept (Zaltrap®), Aldara, Aldesleukin (IL-2, Proleukin or interleukin 2), Alemtuzumab (MabCampath), Alkeran, Amsacrine (Amsidine, m-AMSA), Amsidine, Anastrozole (Arimidex®), Ara C, Aredia, Arimidex, Aromasin, Arsenic trioxide (Trisenox®, ATO), Asparaginase (Crisantaspase®, Erwinase®), Axitinib (Inlyta®), Azacitidine (Vidaza®), BEACOPP, BEAM, Bendamustine (Levact®), Bevacizumab (Avastin), Bexarotene (Targretin®), Bicalutamide (Casodex®), Bleomycin, Bleomycin, etoposide and platinum (BEP), Bortezomib (Velcade®), Bosulif, Bosutinib (Bosulif), Brentuximab (Adcetris®), Brufen, Buserelin (Suprefact®), Busilvex, Busulfan (Myleran, Busilvex), CAPE-OX, CAPOX, CAV, CAVE, CCNU, CHOP, CMF, CMV, CVP, Cabazitaxel (Jevtana®), Cabozantinib (Cometriq®), Caelyx, Calpol, Campto, Capecitabine (Xeloda®), Caprelsa, Carbo MV, CarboTaxol, Carboplatin, Carboplatin and etoposide, Carboplatin and paclitaxel, Carmustine (BCNU, Gliadel®), Casodex, Ceritinib (Zykadia®), Cerubidin, Cetuximab (Erbitux®), ChlVPP, Chlorambucil (Leukeran®), Cisplatin, Cisplatin and Teysuno, Cisplatin and capecitabine (CX), Cisplatin, etoposide and ifosfamide (PEI), Cisplatin, fluorouracil (5-FU) and trastuzumab, Cladribine (Leustat®, LITAK), Clasteon, Clofarabine (Evoltra®), Co-codamol (Kapake®, Solpadol®, Tylex®), Cometriq, Cosmegen, Crisantaspase, Crizotinib (Xalkori®), Cyclophosphamide, Cyclophosphamide, thalidomide and dexamethasone (CTD), Cyprostat,Cyproterone acetate (Cyprostat®), Cytarabine (Ara C, cytosine arabinoside), Cytarabine into spinal fluid, Cytosine arabinoside, DHAP,DTIC, Dabrafenib (Tafinlar®),Dacarbazine (DTIC), Dacogen, Dactinomycin (actinomycin D, Cosmegen®), Dasatinib (Sprycel), Daunorubicin, De Gramont, Decapeptyl SR, Decitabine (Dacogen®), Degarelix (Firmagon®), Denosumab (Prolia®, Xgeva®), Depocyte, Dexamethasone, Diamorphine, Disodium pamidronate, Disprol, Docetaxel (Taxotere®), Docetaxel, cisplatin and fluorouracil (TPF), Doxifos, Doxil, Doxorubicin (Adriamycin), Doxorubicin and ifosfamide (Doxifos), Drogenil, Durogesic, EC, ECF, EOF, EOX, EP, ESHAP, Effentora, Efudix, Eldisine, Eloxatin, Enzalutamide, Epirubicin (Pharmorubicin®), Epirubicin cisplatin and capecitabine (ECX), Epirubicin, carboplatin and capecitabine (ECarboX), Eposin, Erbitux, Eribulin (Halaven®), Erlotinib (Tarceva®), Erwinase, Estracyt, Etopophos, Etoposide (Eposin®, Etopophos®, Vepesid®), Everolimus (Afinitor®), Evoltra, Exemestane (Aromasin®), FAD, FEC, FEC-T chemotherapy, FMD, FOLFIRINOX, FOLFOX, Faslodex, Femara, Fentanyl, Firmagon, Fludara, Fludarabine (Fludara®), Fludarabine, cyclophosphamide and rituximab (FCR), Fluorouracil (5FU), Flutamide, Folinic acid, fluorouracil and irinotecan (FOLFIRI), Fulvestrant (faslodex®), G-CSF, Gefitinib (Iressa), GemCarbo (gemcitabine and carboplatin), GemTaxol, Gemcitabine (Gemzar), Gemcitabine and capecitabine (GemCap), Gemcitabine and cisplatin (GC), Gemcitabine and paclitaxel (GemTaxol®),Gemzar,Giotrif, Gliadel, Glivec, Gonapeptyl, Depot, Goserelin (Zoladex®), Goserelin (Zoladex®, Novgos®), Granulocyte colony stimulating factor (G-CSF), Halaven,Herceptin, Hycamtin, Hydrea, Hydroxycarbamide (Hydrea®), Hydroxyurea, I-DEX, ICE, IL-2, IPE, Ibandronic acid, Ibritumomab (Zevalin®), Ibrutinib (Imbruvica®), Ibuprofen (Brufen®, Nurofen®), Iclusig, Idarubicin (Zavedos®), Idarubicin and dexamethasone, Idelalisib (Zydelig®), Ifosfamide (Mitoxana®), Imatinib (Glivec®), Imiquimod cream (Aldara®), Imnovid, Instanyl, Interferon (Intron A), Interleukin, Intron A, Ipilimumab (Yervoy®), Iressa, Irinotecan (Campto®), Irinotecan and capecitabine (Xeliri®), Irinotecan de Gramont, Irinotecan modified de Gramont, Javlor, Jevtana, Kadcyla, Kapake, Keytruda, Lanreotide (Somatuline®), Lanvis, Lapatinib (Tyverb®), Lenalidomide (Revlimid®), Letrozole (Femara®), Leukeran, Leuprorelin (Prostap®, Lutrate®), Leustat, Levact, Liposomal doxorubicin, Litak, Lomustine (CCNU), Lynparza, Lysodren, MIC, MMM, MPT, MST Continus, MVAC, MVP, MabCampath, Mabthera, Maxtrex, Medroxyprogesterone acetate (Provera), Megace, Megestrol acetate (Megace®), Melphalan (Alkeran®), Mepact, Mercaptopurine (Xaluprine®), Methotrexate (Maxtrex), Methyl prednisolone, Mifamurtide (Mepact®), Mitomycin C, Mitotane, Mitoxana, Mitoxantrone (Mitozantrone®), Morphgesic SR, Morphine, Myleran, Myocet, Nab-paclitaxel, Nab-paclitaxel (Abraxane®), Navelbine, Nelarabine (Atriance®), Nexavar, Nilotinib (Tasigna®), Nintedanib (Vargatef®), Nipent, Nivolumab (Opdivo®), Novgos, Nurofen, Obinutuzumab (Gazyvaro®), Octreotide, Ofatumumab (Arzerra®), Olaparib (Lynparza®), Oncovin, Onkotrone, Opdivo, Oramorph, Oxaliplatin (Eloxatin), Oxaliplatin and capecitabine (Xelox®), PAD, PC (paclitaxel and carboplatin, CarboTaxol), PCV, PE, PMitCEBO, POMB/ACE, Paclitaxel (Taxol®), Paclitaxel and carboplatin, Pamidronate, Panadol, Panitumumab (Vectibix®), Paracetamol, Pazopanib (Votrient®), Pembrolizumab (Keytruda), Pemetrexed (Alimta®), Pemetrexed and carboplatin, Pemetrexed and cisplatin, Pentostatin (Nipent®), Perjeta, Pertuzumab (Perjeta®), Pixantrone (Pixuvri®), Pixuvri, Pomalidomide (Imnovid®), Ponatinib, Potactasol, Prednisolone, Procarbazine, Proleukin, Prolia, Prostap, Provera, Purinethol, R-CHOP, R-CVP, R-DHAP, R-ESHAP, R-GCVP, RICE, Raloxifene, Raltitrexed (Tomudex®), Regorafenib (Stivarga®), Revlimid, Rituximab, (Mabthera®), Sevredol, Sodium clodronate (Bonefos®, Clasteon®, Loron®), Solpadol, Sorafenib (Nexavar®), Steroids (dexamethasone, prednisolone, methylprednisolone), Streptozocin (Zanosar®), Sunitinib (Sutent®), Sutent, TAC, TIP, Tafinlar, Tamoxifen, Tarceva, Targretin, Tasigna, Taxol, Taxotere, Taxotere and cyclophosphamide (TC), Temodal, Temozolomide, (Temodal®), Temsirolimus (Torisel®), Tepadina, Teysuno, Thalidomide, Thiotepa (Tepadina®), Tioguanine (thioguanine®, 6-TG, 6-tioguanine), Tomudex, Topotecan (Hycamtin, Potactasol), Torisel, Trabectedin (Yondelis), Trastuzumab (Herceptin®), Trastuzumab emtansine (Kadcyla®), Treosulfan, Tretinoin (Vesanoid®, ATRA), Triptorelin (Decapeptyl SR®, Gonapeptyl Depot®), Trisenox, Tylex, Tyverb,VIDE, Vandetanib (Caprelsa®), Vargatef, VeIP, Vectibix, Velbe, Velcade, Vemurafenib (Zelboraf®), Vepesid, Vesanoid, Vidaza, Vinblastine (Velbe®), Vincristine, Vincristine, actinomycin D (dactinomycin®) and cyclophosphamide (VAC), Vincristine, actinomycin and ifosfamide (VAI), Vincristine, doxorubicin and dexamethasone (VAD), Vindesine (Eldisine®), Vinflunine (Javlor®), Vinorelbine (Navelbine®),Vismodegib (Erivedge®), Votrient, XELOX, Xalkori, Xeloda, Xgeva, Xtandi, Yervoy, Yondelis, Z-DEX, Zaltrap, Zanosar, Zavedos, Zelboraf, Zevalin, Zoladex (breast cancer), Zoladex (prostate cancer), Zoledronic acid (Zometa®), Zometa, Zomorph, Zydelig, Zytiga.

According to a particular embodiment, a co-crystal of 8-chloro-N-(4-(trifluoromethoxy)phenyl)quinolin-2-amine as defined in the present invention, or a pharmaceutically acceptable salt of 8-chloro-N-(4-(trifluoromethoxy)phenyl)quinolin-2-amine including a solvate and/or a hydrate thereof as defined in the present invention, as described herein, can be combined with various chemotherapies, immunotherapy (e.g. check-point inhibitors, monoclonal antibodies), anti-tumoral vaccines, RNA vaccines, magnetic particles, intravascular microrobots, radiotherapy, surgery, ultrasounds or other anti-tumoral therapies.

Therefore, the present invention further provides a co-crystal of 8-chloro-N-(4-(trifluoromethoxy)phenyl)quinolin-2-amine as defined in the present invention, a pharmaceutically acceptable salt of 8-chloro-N-(4-(trifluoromethoxy)phenyl)quinolin-2-amine including a solvate and/or a hydrate thereof as defined in the present invention, or a pharmaceutical composition as defined in the present invention for use as an antitumor agent intended for patients who are also treated with anyone of immunotherapy, anti-tumoral vaccines, RNA vaccines, radiotherapy, surgery, ultrasounds or other anti-tumoral therapies.

According to one embodiment, the present invention relates to the co-crystals of 8-chloro-N-(4-(trifluoromethoxy)phenyl)quinolin-2-amine for use as defined above, the pharmaceutically acceptable salts of 8-chloro-N-(4-(trifluoromethoxy)phenyl)quinolin-2-amine for use as defined above, or the pharmaceutical composition for use as defined above, wherein the level of a co-crystal of 8-chloro-N-(4-(trifluoromethoxy)phenyl)quinolin-2-amine as defined above or of a pharmaceutically acceptable salt of 8-chloro-N-(4-(trifluoromethoxy)phenyl)quinolin-2-amine as defined above, in a blood, plasma, tissue, saliva, and/or serum sample of the patient is measured during the use.

According to another embodiment, the present invention also relates to the co-crystals of 8-chloro-N-(4-(trifluoromethoxy)phenyl)quinolin-2-amine for use as defined above, the pharmaceutically acceptable salts of 8-chloro-N-(4-(trifluoromethoxy)phenyl)quinolin-2-amine for use as defined above, or the pharmaceutical composition for use as defined above, wherein the use is intended for a patient whose level of a co-crystal of 8-chloro-N-(4-(trifluoromethoxy)phenyl)quinolin-2-amine as defined above or of a pharmaceutically acceptable salt of 8-chloro-N-(4-(trifluoromethoxy)phenyl)quinolin-2-amine as defined above, in a blood, plasma, tissue, saliva, and/or serum sample of the patient is measured during the use.

According to still another embodiment, the present invention also relates to the co-crystals of 8-chloro-N-(4-(trifluoromethoxy)phenyl)quinolin-2-amine for use as defined above, the pharmaceutically acceptable salts of 8-chloro-N-(4-(trifluoromethoxy)phenyl)quinolin-2-amine for use as defined above, or the pharmaceutical composition for use as defined above, wherein a presence and/or expression level of miR-124 in a blood and/or tissue sample of the patient, is measured prior to and/or during the use, in particular for monitoring efficacy of the use and/or response to the use.

Hereinafter, the present invention will be described in more detail with reference to the following examples. These examples are provided to illustrate the present invention and should not be construed as limiting the scope and spirit of the present invention.

### EXAMPLES

### MATERIAL AND METHODS

### I. Differential Scanning Calorimetry (DSC)

- TA INSTRUMENTS Q200
- Aluminium sealed sample pan (Not hermetic)
- Atmosphere : Nitrogen
- Heating rate : 10K/min
- Data treatment: UNIVERSAL ANALYSIS 2000 v4.3

The samples were analyzed by DSC from 0°C temperature up to 300°C.

### II. X-Ray Powder Diffraction (XRPD)

- Diffractometers Bruker D8 Advance;
- Cupper anti cathode, tension 35 KV, intensity 40 mA
- Bragg-Brentano configuration, fixed sample
- Range of analysis : 2° to 40°
- Step increment: 0.04°
- Measuring time by step: 1s
- Experimental treatment of the data by the EVA software (v 11.0)

The X-Ray peak positions and intensities are extracted from the analyzed samples.

### Example 1: Preparation of a co-crystal of 8-Chloro-N-(4-(trifluoromethoxy)phenyl)quinolin-2-amine : L-Proline

6.6 mg of 8-chloro-N-(4-(trifluoromethoxy)phenyl)quinolin-2-amine were mixed with 1.6mg of L-Proline as a co-former and 2µl of acetonitrile and then the obtained mixture was grounded at 20hz for 45 min using a Retsch MM200 instrument to obtain 7mg of a co-crystal of 8-Chloro-N-(4-(trifluoromethoxy)phenyl)quinolin-2-amine : L-Proline.

The 8-chloro-N-(4-(trifluoromethoxy)phenyl)quinolin-2-amine : L-Proline co-crystal was characterized by X-Ray Powder Diffraction (XRPD) to confirm co-crystal formation and by Differential Scanning Calorimetry (DSC) to determine the thermal behavior of new solid forms.

More particularly, this 8-chloro-N-(4-(trifluoromethoxy)phenyl)quinolin-2-amine : L-Proline co-crystal has a powder X-ray diffractogram displaying peaks expressed as degree 2-Theta angle at 16.5; 20.6; 21.4; and 22.1 (each time ±0.2), and which may optionally further show the following additional peaks expressed as degree 2-Theta angle: 11.0; 15.9; 18.3; and 19.4 (each time ±0.2); and even optionally further the following additional peaks expressed as degree 2-Theta angle 6.1; 12.2; 12.6; 13.3; 13.7; 15.4; 17.3 and 22.4 (each time ±0.2), optionally further characterized by a powder X-ray diffractogram as illustrated in figure 1 and/or having a single endotherm with an onset temperature of 172.0°C (±2°C).

The same example was conducted except that acetonitrile was replaced by methanol as a polar organic solvent and co-crystal of 8-Chloro-N-(4-(trifluoromethoxy)phenyl)quinolin-2-amine : L-Proline co-crystal is obtained.

### Example 2: Preparation of a co-crystal of 8-Chloro-N-(4-(trifluoromethoxy)phenyl)quinolin-2-amine : Gentisic acid

8.9 mg of 8-chloro-N-(4-(trifluoromethoxy)phenyl)quinolin-2-amine were mixed with 2.3mg of Gentisic acid as a co-former and 2µl of acetonitrile and then the obtained mixture was grounded at 20hz for 45 min using a Retsch MM200 instrument to obtain 7mg of a co-crystal of 8-Chloro-N-(4-(trifluoromethoxy)phenyl)quinolin-2-amine : Gentisic acid.

The 8-chloro-N-(4-(trifluoromethoxy)phenyl)quinolin-2-amine : Gentisic acid co-crystal was characterized by X-Ray Powder Diffraction (XRPD) to confirm co-crystal formation and by Differential Scanning Calorimetry (DSC) to determine the thermal behavior of new solid forms.

More particularly, this 8-chloro-N-(4-(trifluoromethoxy)phenyl)quinolin-2-amine : Gentisic acid co-crystal has a powder X-ray diffractogram displaying peaks expressed as degree 2-Theta angle at 7.9; 14.0; 15.2; and 25.2 (each time ±0.2), and which may optionally further show the following additional peaks expressed as degree 2-Theta angle: 15.8; 16.9; 18.5; 19.9; 20.3; 23.0 and 24.7 (each time ±0.2); and even optionally further the following additional peaks expressed as degree 2-Theta angle: 7.6; 14.7; 16.1; 19.7; 21.6; 22.0; 22.3; 23.7; and 24.0 (each time ±0.2), optionally further characterized by a powder X-ray diffractogram as illustrated in figure 2 and/or having a single endotherm with an onset temperature of 133.0°C (±2°C).

The same example was conducted except that acetonitrile was replaced by methanol as a polar organic solvent and co-crystal of 8-chloro-N-(4-(trifluoromethoxy)phenyl)quinolin-2-amine : Gentisic acid is obtained.

### Example 3: Preparation of a co-crystal of 8-Chloro-N-(4-(trifluoromethoxy)phenyl)quinolin-2-amine : Malonic acid

5.8 mg of 8-chloro-N-(4-(trifluoromethoxy)phenyl)quinolin-2-amine were mixed with 1.5mg of Malonic acid as a co-former and 2µl of acetonitrile and then the obtained mixture was grounded at 20hz for 45 min using a Retsch MM200 instrument to obtain 7mg of a co-crystal of 8-chloro-N-(4-(trifluoromethoxy)phenyl)quinolin-2-amine : Malonic acid

The 8-chloro-N-(4-(trifluoromethoxy)phenyl)quinolin-2-amine : Malonic acid co-crystal was characterized by X-Ray Powder Diffraction (XRPD) to confirm co-crystal formation and by Differential Scanning Calorimetry (DSC) to determine the thermal behavior of new solid forms.

More particularly, this 8-chloro-N-(4-(trifluoromethoxy)phenyl)quinolin-2-amine : Malonic acid co-crystal has a powder X-ray diffractogram displaying peaks expressed as degree 2-Theta angle at 9.5; 12.2; 15.8; 17.3; 19.7; 22.8; 24.8; and 25.6 (each time ±0.2), and which may optionally further show the following additional peaks expressed as degree 2-Theta angle: 19.0; 21.4; 24.6; 26.8; 27.6; and 29.9 (each time ±0.2); and even optionally further the following additional peaks expressed as degree 2-Theta angle 16.8; 17.8; 20.9; 23.8; 28.0; and 29.6 (each time ±0.2), optionally further characterized by a powder X-ray diffractogram as illustrated in figure 3 and/or having a single endotherm with an onset temperature of 109.0°C (±2°C).

The same example was conducted except that acetonitrile was replaced by methanol as a polar organic solvent and co-crystal of 8-chloro-N-(4-(trifluoromethoxy)phenyl)quinolin-2-amine : Malonic acid is obtained.

### Example 4: Preparation of a co-crystal of 8-Chloro-N-(4-(trifluoromethoxy)phenyl)quinolin-2-amine : 4,4'-Bipyridine

8.6 mg of 8-chloro-N-(4-(trifluoromethoxy)phenyl)quinolin-2-amine were mixed with 2.4mg of 4, 4'-Bipyridine as a co-former and 2µl of acetonitrile and then the obtained mixture was grounded at 20hz for 45 min using a Retsch MM200 instrument to obtain 7mg of a co-crystal of 8-Chloro-N-(4-(trifluoromethoxy)phenyl)quinolin-2-amine : of 4, 4'-Bipyridine.

The 8-chloro-N-(4-(trifluoromethoxy)phenyl)quinolin-2-amine : 4, 4'-Bipyridine co-crystal was characterized by X-Ray Powder Diffraction (XRPD) to confirm co-crystal formation and by Differential Scanning Calorimetry (DSC) to determine the thermal behavior of new solid forms.

More particularly, this 8-chloro-N-(4-(trifluoromethoxy)phenyl)quinolin-2-amine : 4, 4'-Bipyridine co-crystal has a powder X-ray diffractogram displaying peaks expressed as degree 2-Theta angle at 12.0; 19.2; 21.2; and 24.3 (each time ±0.2), and which may optionally further show the following additional peaks expressed as degree 2-Theta angle: 16.0; 17.0; 17.8; 20.3; 22.5; and 22.7 (each time ±0.2); and even optionally further the following additional peaks expressed as degree 2-Theta angle: 8.5; 13.0; 15.7; 16.7; 20.9; 22.0; 23.1; 23.6 and 24.7 (each time ±0.2), optionally further characterized by a powder X-ray diffractogram as illustrated in figure 4 and/or having a single endotherm with an onset temperature of 127.0°C (±2°C).

The same example was conducted except that acetonitrile was replaced by methanol as a polar organic solvent and co-crystal of 8-chloro-N-(4-(trifluoromethoxy)phenyl)quinolin-2-amine : 4, 4'-Bipyridine is obtained.

### Example 5: Preparation of anhydrous crystalline ABX464 hemi-napadisylate salt

11.9mg of 8-chloro-N-(4-(trifluoromethoxy)phenyl)quinolin-2-amine were dissolved in 500µl of methanol. To this mixture were added 175µl of naphthalene1,5disulfonic acid 0.1M (counter ion) in ethanol so as to obtain a ABX464: counter ion molar ratio of 2:1.
The mixture preparation was magnetic stirred at room temperature (25°C) for ½ hour. Solvents were evaporated at room temperature (25°C) under N2 gas flow. Acetone was then added as crystallization solvent (1180 µl of acetone) into the tube. The tube was then hermetically closed to prevent evaporation of the crystallization solvent and heated 1h at 60°C while the solution was stirred by a magnetic bare. To induce crystallization of products, the tube was then cooled at a 0.1°C/min rate and to 5°C while the solution was stirred by a magnetic bare. The obtained suspension was then filtered under 0.2µm mesh and then drying under vacuum at 40°C. 8 mg of an anhydrous crystalline ABX464 hemi-napadisylate salt is thus obtained.

As already mentioned in the present text, this salt has a powder X-ray diffractogram displaying peaks expressed as degree 2-Theta angle at 9.8; 16.4; 18.2; 20.1; 21.2; 21.6; 23.5 and 26.3 (each time ±0.2), and optionally further shows the following additional peaks expressed as degree 2-Theta angle: 12.4; 13.1; 17.8; 20.9; 22.6; 24.5; 24.7; 25.2; and 25.9 (each time ±0.2); and even optionally further the following additional peaks expressed as degree 2-Theta angle: 8.8; 13.3; 15.1; 17.2; 17.5; 19.4; 19.5; and 19.8 (each time ±0.2), as illustrated in figure 5 (powder X-ray diffractogram) and/or having a single endotherm with an onset temperature of 269.0°C (±2°C).

A characteristic X-ray powder diffractogram of an anhydrous crystalline ABX464 hemi-napadisylate salt is thus given in figure 5.

### Example 6: Preparation of anhydrous crystalline ABX464 esylate salt

11.4mg of 8-chloro-N-(4-(trifluoromethoxy)phenyl)quinolin-2-amine were dissolved in 500µl of methanol. To this mixture were added 337µl of ethane sulfonic acid 0.1M (counter ion) in water/ethanol (1/10) so as to obtain a ABX464: counter ion molar ratio of 1:1.

The mixture preparation was magnetic stirred at room temperature (25°C) for ½ hour. Solvents were evaporated at room temperature (25°C) under N2 gas flow. Ethyl Acetate was then added as crystallization solvent (480 µl of Ethyl Acetate) into the tube. The tube was then hermetically closed to prevent evaporation of the crystallization solvent and heated 1h at 60°C while the solution was stirred by a magnetic bare. To induce crystallization of products, the tube was then cooled at a 0.1°C/min rate and to 5°C while the solution was stirred by a magnetic bare. The obtained solution was then evaporated under N2 gas flow at room temperature. 12 mg of an anhydrous crystalline ABX464 esylate salt is thus obtained.

As already mentioned in the present text, this salt has a powder X-ray diffractogram displaying peaks expressed as degree 2-Theta angle at 12.2; and 22.2 (each time ±0.2), and optionally further shows the following additional peaks expressed as degree 2-Theta angle: 6.2; 12.9; 13.1; 15.3; 16.3; 18.2; 18.6; 19.5; 20.0; and 20.7 (each time ±0.2); and even optionally further the following additional peaks expressed as degree 2-Theta angle: 10.1; 15.8; 17.7; 17.9; 20.3; and 21.4 (each time ±0.2), as illustrated in figure 6 (powder X-ray diffractogram) and/or having a single endotherm with an onset temperature of 108.0°C (±2°C).

A characteristic X-ray powder diffractogram of anhydrous crystalline ABX464 esylate salt is thus given in figure 6.

### Example 7: Preparation of crystalline hemi-THF (tetrahydrofuran) solvate of ABX464 hemi-napadisvlate salt

10.6mg of 8-chloro-N-(4-(trifluoromethoxy)phenyl)quinolin-2-amine were dissolved in 500µl of methanol. To this mixture were added 313µl of naphthalene1,5disulfonic acid 0.1M (counter ion) in ethanol so as to obtain a ABX464: counter ion molar ratio of 2:1.

The mixture preparation was magnetic stirred at room temperature (25°C) for ½ hour. Solvents were evaporated at room temperature (25°C) under N2 gas flow. THF was then added as crystallization solvent (1030 µl of THF) into the tube. The tube was then hermetically closed to prevent evaporation of the crystallization solvent and heated 1h at 60°C while the solution was stirred by a magnetic bare. To induce crystallization of products, the tube was then cooled at a 0.1°C/min rate and to 5°C while the solution was stirred by a magnetic bare. The obtained suspension was then filtered under 0.2µm mesh and then drying under vacuum at 40°C. 8 mg of a crystalline hemi-THF (tetrahydrofuran) solvate of ABX464 hemi-napadisylate salt is thus obtained.

As already mentioned in the present text, this salt has a powder X-ray diffractogram displaying peaks expressed as degree 2-Theta angle at 8.4; 12.3; 14.0; 19.2; 21.3; 22.6 and 24.6 (each time ±0.2), and optionally further shows the following additional peaks expressed as degree 2-Theta angle: 9.6; 13.0; 13.5; 14.8; 17.2; 17.8; 23.4; 24.1; 24.9 and 25.2 (each time ±0.2); and even optionally further the following additional peaks expressed as degree 2-Theta angle: 16.7; 18.1; 18.8; 19.5; 20.9 and 22.3 (each time ±0.2), as illustrated in figure 7 (powder X-ray diffractogram) and/or having a single endotherm with an onset temperature of 172.0°C (±2°C).

A characteristic X-ray powder diffractogram of a crystalline hemi-THF solvate of ABX464 hemi-napadisylate salt is thus given in figure 7.

### Example 8: Pharmaceutical compositions under the form of a capsule in accordance with the invention comprising a co-crystal of ABX464 as defined in the invention

The following capsule was prepared with the ingredients in the respective amounts as specified below in the table 8.

**Table 8**

| **Ingredients** | **Function** | **Amount (in mg) / unit** |
|---|---|---|
| 8-chloro-N-(4-(trifluoromethoxy)phenyl)quinolin-2-amine : 4, 4'-Bipyridine co-crystal as prepared according to example 4 | Active ingredient | 20.00 equivalents ABX464 |
| MANNITOL | Filler | 120.00 |
| PREGELATINIZED STARCH | Binder | 10.00 |
| TALC | Glidant | 0.85 |
| ZINC STEARATE | Lubricant | 0.85 |
| White opaque hard gelatin capsule, size 1 | Capsule shell | 1 unit |
| sold by Capsugel Belgium NV | | |
| (Body composition: 2% TiO₂ and qsp 100% gelatin (bovine and/or porcine origin) | | |
| Cap composition: 2% TiO₂ and qsp 100% gelatin (bovine and/or porcine origin)). | | |

The pharmaceutical compositions in accordance with the invention are useful in the prevention and/or treatment of inflammatory diseases such as Inflammatory Bowel Disease, Rheumatoid Arthritis, pulmonary arterial hypertension, NASH (nonalcoholic steatohepatitis) and Multiple Sclerosis, diseases caused by viruses and/or cancer or dysplasia.

### Example 9: Pharmaceutical composition under the form of a capsule in accordance with the invention comprising a pharmaceutically acceptable salt of ABX464 as defined in the invention

The following capsule was prepared with the ingredients in the respective amounts as specified below in the table 9.

**Table 9**

| **Ingredients** | **Function** | **Amount (in mg) / unit** |
|---|---|---|
| Crystalline hemi-THF solvate of ABX464 hemi-napadisylate salt as prepared according to example 7 | Active ingredient | 20.00 equivalents ABX464 |
| MANNITOL | Filler | 120.00 |
| PREGELATINIZED STARCH | Binder | 10.00 |
| TALC | Glidant | 0.85 |
| ZINC STEARATE | Lubricant | 0.85 |
| White opaque hard gelatin capsule, size 1 | Capsule shell | 1 unit |
| sold by Capsugel Belgium NV | | |
| (Body composition: 2% TiO₂ and qsp 100% gelatin (bovine and/or porcine origin) | | |
| Cap composition: 2% TiO₂ and qsp 100% gelatin (bovine and/or porcine origin)). | | |

The pharmaceutical compositions in accordance with the invention are useful in the treatment and/or prevention of inflammatory diseases such as Inflammatory Bowel Disease, Rheumatoid Arthritis, pulmonary arterial hypertension, NASH and Multiple Sclerosis, diseases caused by viruses and/or cancer or dysplasia.

## Claims

1. Co-crystals of 8-chloro-N-(4-(trifluoromethoxy)phenyl)quinolin-2-amine which are chosen among:
- 8-Chloro-N-(4-(trifluoromethoxy)phenyl)quinolin-2-amine : L-Proline having a powder X-ray diffractogram displaying peaks expressed as degree 2-Theta angle at 16.5; 20.6; 21.4; and 22.1 (each time ±0.2), and which may optionally further show the following additional peaks expressed as degree 2-Theta angle: 11.0; 15.9; 18.3; and 19.4 (each time ±0.2); and even optionally further the following additional peaks expressed as degree 2-Theta angle 6.1; 12.2; 12.6; 13.3; 13.7; 15.4; 17.3 and 22.4 (each time ±0.2), optionally further **characterized by** a powder X-ray diffractogram as illustrated in figure 1 and/or having a single endotherm with an onset temperature of 172.0°C (±2°C);
- 8-Chloro-N-(4-(trifluoromethoxy)phenyl)quinolin-2-amine :Gentisic acid having a powder X-ray diffractogram displaying peaks expressed as degree 2-Theta angle at 7.9; 14.0; 15.2; and 25.2 (each time ±0.2), and which may optionally further show the following additional peaks expressed as degree 2-Theta angle: 15.8; 16.9; 18.5; 19.9; 20.3; 23.0 and 24.7 (each time ±0.2); and even optionally further the following additional peaks expressed as degree 2-Theta angle: 7.6; 14.7; 16.1; 19.7; 21.6; 22.0; 22.3; 23.7; and 24.0 (each time ±0.2), optionally further **characterized by** a powder X-ray diffractogram as illustrated in figure 2 and/or having a single endotherm with an onset temperature of 133.0°C (±2°C);
- 8-Chloro-N-(4-(trifluoromethoxy)phenyl)quinolin-2-amine : Malonic acid having a powder X-ray diffractogram displaying peaks expressed as degree 2-Theta angle at 9.5; 12.2; 15.8; 17.3; 19.7; 22.8; 24.8; and 25.6 (each time ±0.2), and which may optionally further show the following additional peaks expressed as degree 2-Theta angle: 19.0; 21.4; 24.6; 26.8; 27.6; and 29.9 (each time ±0.2); and even optionally further the following additional peaks expressed as degree 2-Theta angle 16.8; 17.8; 20.9; 23.8; 28.0; and 29.6 (each time ±0.2), optionally further **characterized by** a powder X-ray diffractogram as illustrated in figure 3 and/or having a single endotherm with an onset temperature of 109.0°C (±2°C); and
- 8-Chloro-N-(4-(trifluoromethoxy)phenyl)quinolin-2-amine : 4, 4'-Bipyridine having a powder X-ray diffractogram displaying peaks expressed as degree 2-Theta angle at 12.0; 19.2; 21.2; and 24.3 (each time ±0.2), and which may optionally further show the following additional peaks expressed as degree 2-Theta angle: 16.0; 17.0; 17.8; 20.3; 22.5; and 22.7 (each time ±0.2); and even optionally further the following additional peaks expressed as degree 2-Theta angle: 8.5; 13.0; 15.7; 16.7; 20.9; 22.0; 23.1; 23.6 and 24.7 (each time ±0.2), optionally further **characterized by** a powder X-ray diffractogram as illustrated in figure 4 and/or having a single endotherm with an onset temperature of 127.0°C (±2°C).

2. Method for preparing a co-crystal of 8-chloro-N-(4-(trifluoromethoxy)phenyl)quinolin-2-amine as defined in claim 1 which comprises the following steps:
a) dissolving ABX464 in a solvent or in a mixture of solvents;
b) adding to the thus obtained mixture of step a) a co-former which may be itself already dissolved in a solvent or in a mixture of solvents and which is selected from L-proline, malonic acid, gentisic acid and 4,4'-bipyridine so as to obtain a ABX464 : co-former in molar ratio comprised between 3:1 and 1:2, particularly between 5:2 and 1:2; more particularly between 2:1 and 1:2, and still more particularly is of 2:1 or 1:1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent or a mixture of solvents,
e) applying a temperature program;
f) optionally filtrating; and
g) then optionally drying at a temperature comprised between room temperature and 60°C in order to obtain the desired co-crystal of ABX464;
or which comprises the following steps:
- a') physically mixing 8-chloro-N-(4-(trifluoromethoxy)phenyl)quinolin-2-amine with a co-former chosen among L-Proline, Gentisic acid, Malonic acid and 4, 4'-Bipyridine at a molar ratio comprised between 2:1 and 1:2, particularly at a 1:1 molar ratio, in a suitable solvent or mixtures of solvents; and
- b') grinding of the physical mixture thus obtained from step a), in presence of one drop of solvent or of mixtures of solvents to obtain the co-crystal.

3. The method according to claim 2, wherein the solvent(s) of step a), step b) and/or step d) or of step a') and / or step b') is(are) any solvent conventionally used in the crystallization step, particularly is(are) organic solvents, more particularly is(are) selected from a C₁-C₆ aliphatic alcohol, methyl ethyl ketone (also named butanone or MEK), cyclohexane, alkane such as heptane, methylene chloride, chloroform, formic acid, DMSO, 1-methyl-2-pyrrolidone, acetone, acetonitrile, tetrahydrofuran (THF), diethyl ether, dioxane, toluene, ethyl acetate, optionally in admixture with water, and mixtures thereof, still more particularly selected from a C₁-C₆ aliphatic alcohol, a mixture of H₂O/ C₁-C₆ aliphatic alcohol, acetonitrile, and mixtures thereof, even more particularly selected from methanol, ethanol, isopropanol, H₂O/methanol, H₂O/ethanol, acetonitrile, and mixtures thereof.

4. A pharmaceutically acceptable salt of 8-chloro-N-(4-(trifluoromethoxy)phenyl)quinolin-2-amine including a solvate and/or a hydrate thereof which is selected from lactate, oleate, oxalate, palmitate, stearate, valerate, pantothenate, picrate, butyrate, malonate, succinate, bitartrate, malate, mandelate, benzoate, edetate, gluceptate, gluconate, lactobionate, salicylate, disalicylate, mucate, pamoate, adipate, alginate, aspartate, camphorate, cyclopentaneproprionate, digluconate, glucoheptonate, heptanoate, hexanoate, laurate, nicotinate, pamoate, pivalate, propionate, undecanoate and the like, phosphate and the like, camphorsulfonate, 2-hydroxy-ethanesulfonate, estolate, napsylate, esylate, napadisylate, dodecylsulfate and the like, perchloric acid, boric acid, glycerophosphoric acid, nitric acid, persulfuric acid and the like, particularly is selected from esylate and napadisylate, more particularly is selected from anhydrous crystalline ABX464 hemi-napadisylate salt, anhydrous crystalline ABX464 esylate salt, and crystalline hemi-THF solvate of ABX464 hemi-napadisylate.

5. The pharmaceutically acceptable salt of 8-chloro-N-(4-(trifluoromethoxy)phenyl)quinolin-2-amine including a solvate and/or a hydrate thereof according to claim 4 which is chosen among:
- anhydrous crystalline ABX464 hemi-napadisylate salt having a powder X-ray diffractogram displaying peaks expressed as degree 2-Theta angle at 9.8; 16.4; 18.2; 20.1; 21.2; 21.6; 23.5 and 26.3 (each time ±0.2), and optionally further shows the following additional peaks expressed as degree 2-Theta angle: 12.4; 13.1; 17.8; 20.9; 22.6; 24.5; 24.7; 25.2; and 25.9 (each time ±0.2); and even optionally further the following additional peaks expressed as degree 2-Theta angle: 8.8; 13.3; 15.1; 17.2; 17.5; 19.4; 19.5; and 19.8 (each time ±0.2), as illustrated in figure 5 (powder X-ray diffractogram) and/or having a single endotherm with an onset temperature of 269.0°C (±2°C);
- anhydrous crystalline ABX464 esylate salt having a powder X-ray diffractogram displaying peaks expressed as degree 2-Theta angle at 12.2; and 22.2 (each time ±0.2), and optionally further shows the following additional peaks expressed as degree 2-Theta angle: 6.2; 12.9; 13.1; 15.3; 16.3; 18.2; 18.6; 19.5; 20.0; and 20.7 (each time ±0.2); and even optionally further the following additional peaks expressed as degree 2-Theta angle: 10.1; 15.8; 17.7; 17.9; 20.3; and 21.4 (each time ±0.2), as illustrated in figure 6 (powder X-ray diffractogram) and / or having a single endotherm with an onset temperature of 108.0°C (±2°C); and
- crystalline hemi-THF solvate of ABX464 hemi-napadisylate salt having a powder X-ray diffractogram displaying peaks expressed as degree 2-Theta angle at 8.4; 12.3; 14.0; 19.2; 21.3; 22.6 and 24.6 (each time ±0.2), and optionally further shows the following additional peaks expressed as degree 2-Theta angle: 9.6; 13.0; 13.5; 14.8; 17.2; 17.8; 23.4; 24.1; 24.9 and 25.2 (each time ±0.2); and even optionally further the following additional peaks expressed as degree 2-Theta angle: 16.7; 18.1; 18.8; 19.5; 20.9 and 22.3 (each time ±0.2), as illustrated in figure 7 (powder X-ray diffractogram) and/or having a single endotherm with an onset temperature of 172.0°C (±2°C).

6. Method for preparing a pharmaceutically acceptable salt of ABX464 including solvate or hydrate thereof as defined in claim 4 or claim 5 which comprises the following steps:
a) dissolving ABX464 in a solvent or in a mixture of solvents;
b) adding to the thus obtained mixture of step a) a counter ion under the form of an acid which may be itself already dissolved in a solvent or in a mixture of solvents so as to obtain a ABX464 : counter ion molar ratio comprised between 3:1 and 1:2, particularly between 5:2 and 1:2; more particularly between 2:1 and 1:2, and still more particularly is of 2:1 or 1 :1;
c) optionally evaporating the solvent(s) at a temperature comprised between 0°C and the boiling point of the selected solvent(s) or mixture of solvent(s) of step a) and step b), particularly between room temperature and 60°C, more particularly between room temperature and 50°C;
d) optionally adding a solvent or a mixture of solvents,
e) applying a temperature program;
f) optionally filtrating; and
g) then optionally drying at a temperature comprised between room temperature and 60°C in order to obtain the desired salt of ABX464.

7. The method according to claim 6, wherein the solvent of step a), step b) and/or step d) is any solvent conventionally used in the crystallization step, particularly is organic solvents, more particularly is selected from a C₁-C₆ aliphatic alcohol, methyl ethyl ketone (also named butanone or MEK), cyclohexane, alkane such as heptane, methylene chloride, chloroform, formic acid, DMSO, 1-methyl-2-pyrrolidone, acetone, acetonitrile, tetrahydrofuran (THF), diethyl ether, dioxane, toluene, ethyl acetate, optionally in admixture with water, and mixtures thereof, still more particularly selected from a C₁-C₆ aliphatic alcohol, a mixture of H₂O/ C₁-C₆ aliphatic alcohol, and mixtures thereof, even more particularly selected from methanol, ethanol, isopropanol, H₂O/methanol, H₂O/ethanol, and mixtures thereof; and/or
the counter ion under the form of an acid of step b) is ethane sulfonic acid or naphthalene 1,5-disulfonic acid.

8. The method according to anyone of claims 2, 3, 6 and 7, wherein the step e) relative to the temperature program includes i) a heating at reflux to a temperature comprised between room temperature and the boiling point of the solvent(s), particularly between room temperature and 60°C and/or ii) a cooling at reflux to a temperature comprised between 0°C and 60°C, particularly between 5°C and 40°C, more particularly between room temperature and 40°C at a rate comprised between 30°C/min and 0.05°C/min, particularly between 10°C/min and 0.05°C/min, more particularly between 5°C/min and 0.05°C/min.

9. The method according to anyone of claims 2, 3, 6, 7 and 8, wherein the step f) regarding the filtration is carried out using conventional glass fibers, conventional cellulosic filter papers, PTFE (polytetrafluoroethylene), or PVDF (polyvinylidene fluoride), particularly cellulosic filter paper with 0.45µm or 0.2µm filtration mesh.

10. The method according to anyone of claims 2, 3, 6, 7, 8, 9 and 10, wherein the step g) regarding the drying is carried out under vacuum at a temperature comprised between 30°C and 60°C, particularly at 40°C or under ambient atmosphere at a temperature comprised between 30°C and 60°C, particularly at 40°C.

11. Pharmaceutical composition comprising at least one of the co-crystals as defined in claim 1 and/or at least one of the pharmaceutically acceptable salts of 8-chloro-N-(4-(trifluoromethoxy)phenyl)quinolin-2-amine including a solvate and/or a hydrate thereof as defined in claim 4 or claim 5, and at least one pharmaceutically acceptable excipient.

12. Pharmaceutical composition according to claim 11, wherein the pharmaceutical composition comprises at least one of the co-crystals as defined in claim 1 and/or at least one of the pharmaceutically acceptable salts of 8-chloro-N-(4-(trifluoromethoxy)phenyl)quinolin-2-amine including a solvate and/or a hydrate thereof as defined in claim 4 or claim 5, as the sole pharmaceutically active ingredient(s).

13. The co-crystals of 8-chloro-N-(4-(trifluoromethoxy)phenyl)quinolin-2-amine as defined in claim 1, the pharmaceutically acceptable salts of 8-chloro-N-(4-(trifluoromethoxy)phenyl)quinolin-2-amine as defined in claim 4 or claim 5, or the pharmaceutical composition as defined in claim 11 or claim 12 for use as a medicament.

14. The co-crystals of 8-chloro-N-(4-(trifluoromethoxy)phenyl)quinolin-2-amine as defined in claim 1, the pharmaceutically acceptable salts of 8-chloro-N-(4-(trifluoromethoxy)phenyl)quinolin-2-amine as defined in claim 4 or claim 5, or the pharmaceutical composition as defined in claim 11 or claim 12 for use in the prevention and/or treatment an inflammatory disease.

15. The co-crystals of 8-chloro-N-(4-(trifluoromethoxy)phenyl)quinolin-2-amine as defined in claim 1, the pharmaceutically acceptable salts of 8-chloro-N-(4-(trifluoromethoxy)phenyl)quinolin-2-amine as defined in claim 4 or claim 5, or the pharmaceutical composition as defined in claim 11 or claim 12 for use in the treatment and /or prevention of cancer.

16. The co-crystals of 8-chloro-N-(4-(trifluoromethoxy)phenyl)quinolin-2-amine, the pharmaceutically acceptable salts of 8-chloro-N-(4-(trifluoromethoxy)phenyl)quinolin-2-amine or the pharmaceutical composition for use according to anyone of claims 13 to 15, wherein a presence and/or expression level of miR-124 in a blood and/or tissue sample of the patient, is measured prior to and/or during the use, in particular for monitoring efficacy of the use and/or response to the use.

17. The co-crystals of 8-chloro-N-(4-(trifluoromethoxy)phenyl)quinolin-2-amine as defined in claim 1, the pharmaceutically acceptable salts of 8-chloro-N-(4-(trifluoromethoxy)phenyl)quinolin-2-amine as defined in claim 4 or claim 5, or the pharmaceutical composition as defined in claim 11 or claim 12, for use in the treatment and/or prevention of diseases caused by viruses, in particular by retroviruses and more particularly by HIV, and more particularly for use for lowering viral load in a patient infected by a virus, in particular HIV, or a virus-related condition, with a long-lasting effect and absence of resistance.

18. The co-crystals of 8-chloro-N-(4-(trifluoromethoxy)phenyl)quinolin-2-amine, the pharmaceutically acceptable salts of 8-chloro-N-(4-(trifluoromethoxy)phenyl)quinolin-2-amine or the pharmaceutical composition for use according to anyone of claims 13 to 17, wherein the level of a co-crystal of 8-chloro-N-(4-(trifluoromethoxy)phenyl)quinolin-2-amine as defined in claim 1, or of a pharmaceutically acceptable salt of 8-chloro-N-(4-(trifluoromethoxy)phenyl)quinolin-2-amine as defined in claim 4 or claim 5, in a blood, plasma, tissue, saliva, and/or serum sample of the patient is measured during the use.
